(19) Europäisches Patentamt
European Patent Office
Office européen des brevets

(11) EP 4 653 443 A1

(12) EUROPEAN PATENT APPLICATION
published in accordance with Art. 153(4) EPC

(43) Date of publication:
**26.11.2025 Bulletin 2025/48**

(21) Application number: **24744285.8**

(22) Date of filing: **17.01.2024**

(51) International Patent Classification (IPC):
***C07D 495/04*** (2006.01) ***C07D 487/14*** (2006.01)
***A61P 31/12*** (2006.01) ***A61P 37/00*** (2006.01)
***A61K 31/517*** (2006.01) ***A61K 31/519*** (2006.01)
***A61P 25/00*** (2006.01)

(52) Cooperative Patent Classification (CPC):
**A61K 31/517; A61K 31/519; A61P 25/00; A61P 31/12; A61P 37/00; C07D 487/14; C07D 495/04**

(86) International application number:
**PCT/CN2024/072704**

(87) International publication number:
**WO 2024/153110 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.01.2023 CN 202310083163**
**20.04.2023 CN 202310430237**

(71) Applicant: **Phil Rivers Technology Co., Ltd**
**Beijing 100190 (CN)**

(72) Inventors:
- **ZHANG, Tingyan**
**Beijing 100190 (CN)**
- **YANG, Jianfei**
**Beijing 100190 (CN)**
- **NIU, Gang**
**Beijing 100190 (CN)**
- **XUE, Yiqing**
**Beijing 100190 (CN)**
- **ZHANG, Chunming**
**Beijing 100190 (CN)**
- **PENG, Zhengshu**
**Beijing 100190 (CN)**

(74) Representative: **Schüssler, Andrea**
**Kanzlei Huber & Schüssler**
**Truderinger Strasse 246**
**81825 München (DE)**

# (54) STEREOISOMER AND DEUTERATED DERIVATIVE OF PYRAZOLOQUINAZOLINE COMPOUND AND USE

(57) The present disclosure provides a stereoisomer, deuterated derivative or stereoisomeric deuterated derivative of a compound represented by formula (I), or a pharmaceutically acceptable salt and solvate thereof, which can be used as a PLKI inhibitor, wherein $R^1$ and $R^2$ are as described herein. Further provided are a pharmaceutical composition comprising the stereoisomer, deuterated derivative or stereoisomeric deuterated derivative, or the pharmaceutically acceptable salt and solvate, and a use in the treatment of diseases and symptoms caused by PLKI activity disorders and/or PLKI-related diseases and symptoms, such as cancer.

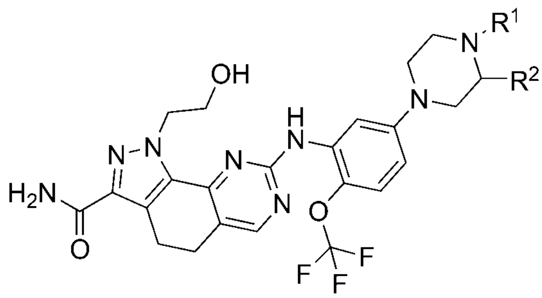

(I)

EP 4 653 443 A1

## Description

This application claims priority to Chinese Patent Application No. 202310083163.X filed on January 17, 2023 and Chinese Patent Application No. 202310430237.2 filed on April 20, 2023, the entire contents of which are incorporated herein by reference.

## TECHNICAL FIELD

The present disclosure relates to stereoisomers, deuterated derivatives or stereoisomeric deuterated derivatives of pyrazoloquinazoline-based compounds, or pharmaceutically acceptable salts, solvates thereof. The present disclosure also relates to pharmaceutical compositions comprising one or more of such stereoisomers and derivatives, or pharmaceutically acceptable salts, solvates thereof, as active ingredients. Furthermore, the present disclosure relates to uses of such stereoisomers and derivatives, or pharmaceutically acceptable salts or solvates thereof, as well as pharmaceutical compositions comprising such stereoisomeric derivatives or pharmaceutically acceptable salts or solvates thereof, as polo-like kinase inhibitors.

## BACKGROUND

Mammalian polo-like kinases (PLKs) consist of a family of five serine/threonine kinases (PLKs1-5) with different functions and expression patterns in mammalian cells. Most PLKs have a C-terminal polo-box domain (PBD) for substrate recognition, and an N-terminal kinase domain for substrate phosphorylation. Among PLKs, PLKl is the most extensively studied family member and plays critical roles in cell cycle progression, especially at the G2-M checkpoint, mitosis, and cytokinesis. PLK1 activity is tightly regulated during cell cycle through interactions with other phosphorylated scaffold proteins.

To date, PLK1 has been extensively studied as an attractive target in cancer therapy, and several small molecule PLK1 inhibitors have been developed over the past decade or more. These inhibitors target both liquid tumors such as leukemia and lymphoma, as well as solid tumors such as pancreatic cancer, breast cancer, and prostate cancer. However, although PLK1 inhibitors have shown anti-tumor effects in preclinical cancer models and have also shown preliminary promising results in clinical trials, they have not yet achieved satisfactory therapeutic effects due to dose-limiting toxicity (Translational Oncology Volume 16, February 2022, 101332). Among clinically investigated ATP-competitive inhibitors primarily targeting PLK1, onvansertib (NMS-P937, NMS1286937) exhibits high selectivity and holds significant clinical development prospects. However, among the onvansertib derivative series (Bioorg Med Chem Lett. 2011 May 15;21(10):2969-74, Bioorg Med Chem Lett. 2010 Nov 15;20(22):6489-94), most derivative molecules show poor oral absorption and bioavailability in mice. The literature (Mol Cancer Ther; 11(4) April 2012, Supplementary table S4) further reveal substantial interspecies variability in onvansertib's absorption and bioavailability, with no discernible pattern, posing challenges for developing selective PLK1 inhibitors.

The researchers of this study unexpectedly discovered that parameters such as oral absorption, bioavailability, and tissue distribution of onvansertib can be effectively modulated by stereocyclization modification, deuteration, and stereocyclization plus deuteration at the N-methylpiperazine of onvansertib. Multiple animal experiments have shown that the modified molecules have lower toxicity and better efficacy than onvansertib, providing a broader selection of effective molecules for different types of diseases.

## SUMMARY OF THE INVENTION

Disclosed herein are stereoisomers, deuterated derivatives or stereoisomeric deuterated derivatives of pyrazoloquinazoline-based compounds as polo-like kinase inhibitors, or pharmaceutically acceptable salts, solvates thereof. Therefore, the compounds disclosed herein are particularly suitable for targeting PLK1, and can be used to treat diseases and conditions caused by dysregulated PLK1 activity and/or diseases and conditions associated with PLK1, including but not limited to leukemia, lymphoma, pancreatic cancer, breast cancer, prostate cancer, lung cancer, ovarian cancer, colorectal cancer, liver cancer, gastric cancer, esophageal cancer, melanoma, multiple myeloma, sepsis, and the like.

In one aspect, the present disclosure relates to a stereoisomer, deuterated derivative or stereoisomeric deuterated derivative of a compound of Formula (I):

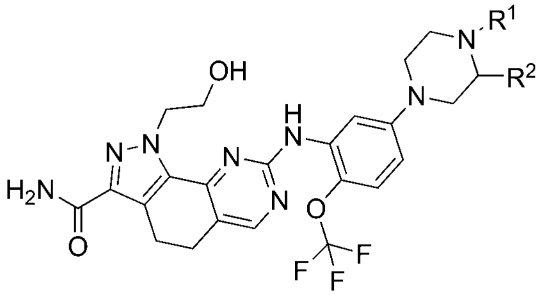

(I)

or a pharmaceutically acceptable salt, solvate thereof,
wherein $R^1$ and $R^2$ are each independently selected from hydrogen, deuterium, deuterated or non-deuterated $C_{1-6}$ alkyl; or $R^1$ and $R^2$ together with the N atom and the C atom to which they are attached form a 5-membered heterocyclic ring;

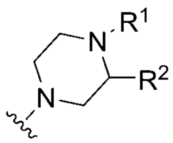

is optionally

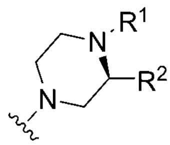

or

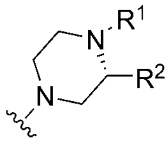

;

and/or

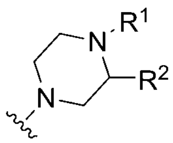

is optionally substituted with one or more deuterium atoms.

In another aspect, the present disclosure relates to a pharmaceutical composition comprising a stereoisomer, deuterated derivative or stereoisomeric deuterated derivative of a compound of Formula (I) as provided herein, or a pharmaceutically acceptable salt, solvate thereof, and a pharmaceutically acceptable carrier or excipient.

In a further aspect, the present disclosure relates to a method for treating diseases and conditions caused by dysregulated PLK1 activity and/or diseases and conditions associated with PLK1 in a subject in need thereof, comprising administering to the subject a therapeutically effective amount of a stereoisomer, deuterated derivative or stereoisomeric deuterated derivative of a compound of Formula (I) as provided herein, or a pharmaceutically acceptable salt, solvate thereof.

In a further aspect, the present disclosure relates to a stereoisomer, deuterated derivative or stereoisomeric deuterated derivative of a compound of Formula (I) as provided herein, or a pharmaceutically acceptable salt, solvate thereof for use in treating diseases and conditions caused by dysregulated PLK1 activity and/or diseases and conditions associated with PLK1.

In a further aspect, the present disclosure relates to the use of a stereoisomer, deuterated derivative or stereoisomeric deuterated derivative of a compound of Formula (I) as provided herein, or a pharmaceutically acceptable salt, solvate thereof, in the preparation of medicaments for treating diseases and conditions caused by dysregulated PLK1 activity and/or diseases and conditions associated with PLK1.

In a further aspect, the present disclosure relates to a kit for treating diseases and conditions caused by dysregulated PLK1 activity and/or diseases and conditions associated with PLK1, the kit comprising: a stereoisomer, deuterated derivative or stereoisomeric deuterated derivative of a compound of Formula (I) as provided herein, or a

pharmaceutically acceptable salt, solvate thereof, or a pharmaceutical composition comprising a stereoisomer, deuterated derivative or stereoisomeric deuterated derivative of a compound of Formula (I) as provided herein, or a pharmaceutically acceptable salt, solvate thereof and a pharmaceutically acceptable carrier or excipient, a container and optionally a package insert or label indicating treatment.

The diseases and conditions caused by dysregulated PLK1 activity and/or diseases and conditions associated with PLK1 include, but are not limited to, leukemia, lymphoma, pancreatic cancer, breast cancer, prostate cancer, lung cancer, ovarian cancer, colorectal cancer, liver cancer, gastric cancer, esophageal cancer, melanoma, multiple myeloma, sepsis, and the like.

## BRIEF DESCRIPTION OF THE DRAWINGS

Figure 1 shows the $IC_{50}$ values of the compounds of the Examples of the present disclosure and a positive control compound for growth inhibition effects on pancreatic cancer cell line (PSN1). Among them, A represents CPD02 of the present disclosure; B represents CPD03 of the present disclosure; C represents the positive control compound NMS-P937; D represents CPD04 of the present disclosure; and E represents CPD06 of the present disclosure.

FIG. 2 shows a graph of tumor growth of tested drugs in a HCT116 subcutaneous tumor xenograft mouse model after treatment initiation with the compounds of the present disclosure.

FIG3 shows a graph of body weight changes of experimental animals in the HCT116 subcutaneous xenograft mouse model after treatment initiation with the compounds of the present disclosure.

FIG. 4 shows a graph of tumor growth of tested drugs in a BxPC-3 subcutaneous xenograft mouse model after treatment initiation with the compounds of the present disclosure.

FIG. 5 shows a graph of body weight changes of experimental animals in the BxPC-3 subcutaneous xenograft mouse model after treatment initiation with the compounds of the present disclosure.

FIG. 6 shows a graph of tumor growth of tested drugs in a PSN1 subcutaneous xenograft mouse model after initiation of treatment with a compound of the present disclosure.

FIG. 7 shows a graph of body weight changes of experimental animals in the PSN1 subcutaneous xenograft mouse model after treatment initiation with the compounds of the present disclosure.

## DETAILED DESCRIPTION

Certain embodiments will now be described in detail, examples of which are illustrated in the accompanying detailed description. Although the enumerated embodiments will be described, it should be understood that they are not intended to limit the present disclosure to these embodiments. On the contrary, the present disclosure is intended to cover all alternatives, modifications and equivalents, which may fall within the scope of the present disclosure as defined by the claims. Those skilled in the art will recognize that many methods and materials similar or equivalent to those described herein that could be used in the implementation of the present disclosure. The present disclosure is by no means limited to the methods and materials described. If one or more incorporated references and similar materials are different or contradictory from the present disclosure, including but not limited to defined terms, term usage, described technology, etc., the present disclosure shall control.

It is to be understood that certain features of the present disclosure, which are described in the context of separate embodiments for clarity, may also be provided in combination in a single embodiment. Conversely, various features of the present disclosure that are described in the context of a single embodiment for conciseness may also be provided separately or in any suitable subcombination.

Provides herein are the following.

Item 1. A stereoisomer, deuterated derivative or stereoisomeric deuterated derivative of a compound of Formula (I):

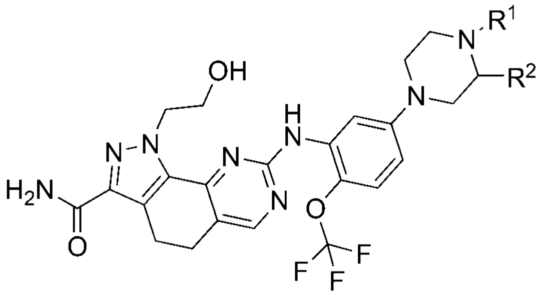

(I)

or a pharmaceutically acceptable salt, solvate thereof,
wherein $R^1$ and $R^2$ are each independently selected from hydrogen, deuterium, deuterated or non-deuterated $C_{1-6}$ alkyl; or $R^1$ and $R^2$ together with the N atom and the C atom to which they are attached form an optionally substituted, deuterated or non-deuterated 5-membered heterocyclic ring;

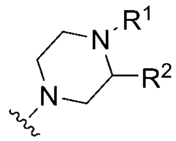

is optionally

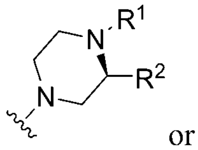

or

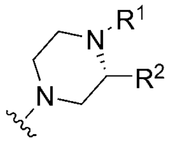

;

and/or

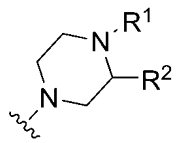

is optionally substituted with one or more deuterium atoms.

Item 2. The stereoisomer, deuterated derivative or stereoisomeric deuterated derivative of a compound of Formula (I), or a pharmaceutically acceptable salt, solvate thereof according to Item 1, wherein

$R^1$ is a deuterated or non-deuterated $C_{1-6}$ alkyl, preferably methyl or deuterated methyl, more preferably perdeuterated methyl;
$R^2$ is hydrogen or deuterium.

Item 3. The stereoisomer, deuterated derivative or stereoisomeric deuterated derivative of a compound of Formula (I), or a pharmaceutically acceptable salt, solvate thereof according to Item 1, wherein

$R^1$ and $R^2$ together with the N atom and C atom to which they are attached form an optionally substituted 5-membered heterocyclic ring as follows:

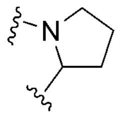

,

wherein the 5-membered heterocyclic ring is optionally substituted with 1-7 deuterium atoms.

Item 4. The stereoisomer, deuterated derivative or stereoisomeric deuterated derivative of a compound of Formula (I), or a pharmaceutically acceptable salt, solvate thereof according to Item 3, wherein

$R^1$ and $R^2$ together with the N atom and C atom to which they are attached form an optionally substituted 5-membered heterocyclic ring selected from:

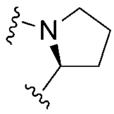

or

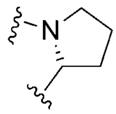

,

wherein the 5-membered heterocyclic ring is optionally substituted with 1-7 deuterium atoms.

Item 5. The stereoisomer, deuterated derivative or stereoisomeric deuterated derivative of a compound of Formula (I), or a pharmaceutically acceptable salt, solvate thereof according to any one of the preceding Items, wherein

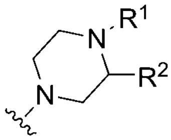

is optionally substituted with 1-8 deuterium atoms.

Item 6. The stereoisomer, deuterated derivative or stereoisomeric deuterated derivative of a compound of Formula (I), or a pharmaceutically acceptable salt, solvate thereof according to Item 1, wherein the stereoisomer, deuterated derivative or stereoisomeric deuterated derivative of a compound of Formula (1) is selected from:

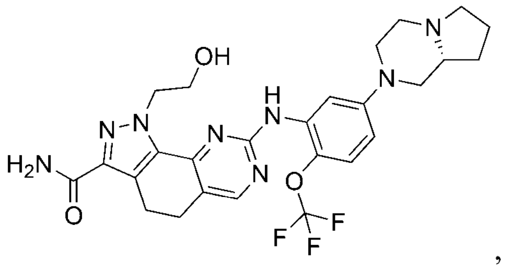

,

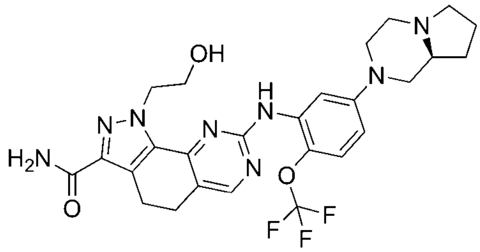

,

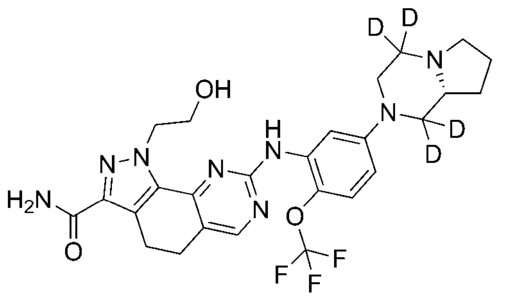

,

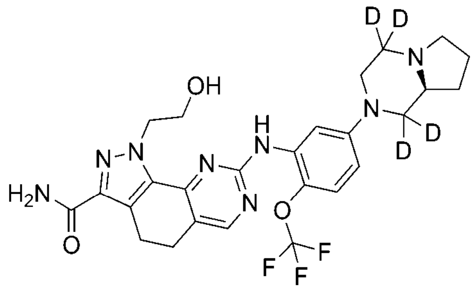

,

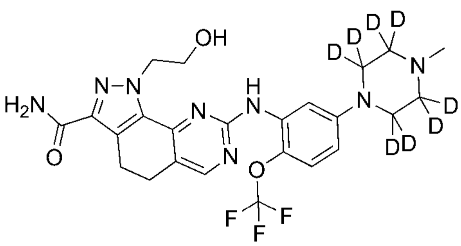

and

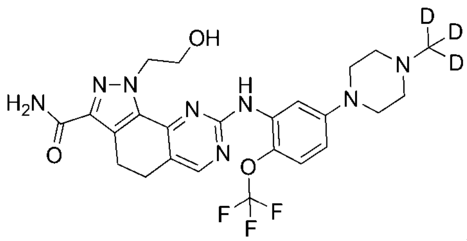

.

Item 7. A pharmaceutical composition, comprising the stereoisomer, deuterated derivative or stereoisomeric deuterated derivative of a compound of Formula (I), or a pharmaceutically acceptable salt, solvate thereof according to any one of Items 1 to 6, and a pharmaceutically acceptable carrier or excipient.
Item 8. A method for treating diseases and conditions caused by dysregulated PLK1 activity and/or diseases and conditions associated with PLK1 in a subject in need thereof, the method comprising administering to the subject a therapeutically effective amount of the stereoisomer, deuterated derivative or stereoisomeric deuterated derivative of a compound of Formula (I), or a pharmaceutically acceptable salt, solvate thereof according to any one of Items 1 to 6.
Item 9. The method according to Item 8, wherein the diseases and conditions caused by dysregulated PLK1 activity and/or diseases and conditions associated with PLK1 are selected from the group consisting of leukemia, lymphoma, pancreatic cancer, breast cancer, prostate cancer, lung cancer, ovarian cancer, colorectal cancer, liver cancer, gastric cancer, esophageal cancer, melanoma, multiple myeloma, and sepsis.
Item 10. The stereoisomer, deuterated derivative or stereoisomeric deuterated derivative of a compound of Formula (I), or a pharmaceutically acceptable salt, solvate thereof according to any one of Items 1 to 6, for use in treating diseases and conditions caused by dysregulated PLK1 activity and/or diseases and conditions associated with PLK1.
Item 11. The stereoisomer, deuterated derivative or stereoisomeric deuterated derivative of a compound of Formula (I), or a pharmaceutically acceptable salt, solvate thereof, for use in treating diseases and conditions caused by dysregulated PLK1 activity and/or diseases and conditions associated with PLK1 according to Item 10, wherein the diseases and conditions caused by dysregulated PLK1 activity and/or diseases and conditions associated with PLK1 are selected from the group consisting of leukemia, lymphoma, pancreatic cancer, breast cancer, prostate cancer, lung cancer, ovarian cancer, colorectal cancer, liver cancer, gastric cancer, esophageal cancer, melanoma, multiple myeloma, and sepsis.
Item 12. Use of the stereoisomer, deuterated derivative or stereoisomeric deuterated derivative of a compound of Formula (I), or a pharmaceutically acceptable salt, solvate thereof according to any one of Items 1 to 6, in the preparation of a medicament for treating diseases and conditions caused by dysregulated PLK1 activity and/or diseases and conditions associated with PLK1.
Item 13. The use according to Item 12, wherein the diseases and conditions caused by dysregulated PLK1 activity and/or diseases and conditions associated with PLK1 are selected from the group consisting of leukemia, lymphoma, pancreatic cancer, breast cancer, prostate cancer, lung cancer, ovarian cancer, colorectal cancer, liver cancer, gastric cancer, esophageal cancer, melanoma, multiple myeloma, and sepsis.
Item 14. A kit for treating diseases and conditions caused by dysregulated PLK1 activity and/or diseases and conditions associated with PLK1, the kit comprising

the stereoisomer, deuterated derivative or stereoisomeric deuterated derivative of a compound of Formula (I), or a pharmaceutically acceptable salt, solvate thereof according to any one of Items 1 to 6, or the pharmaceutical composition according to Item 7;
a container; and
optionally a package insert or label indicating treatment.

Item 15. A kit according to Item 14, wherein the diseases and conditions caused by dysregulated PLK1 activity and/or diseases and conditions associated with PLK1 are selected from the group consisting of leukemia, lymphoma, pancreatic cancer, breast cancer, prostate cancer, lung cancer, ovarian cancer, colorectal cancer, liver cancer, gastric cancer, esophageal cancer, melanoma, multiple myeloma, and sepsis.

## Definitions

Terms used but not defined herein have their ordinary meanings, and the meaning of such terms is independent at each occurrence. However, unless otherwise stated, the following definitions apply throughout the specification and claims.
As used herein, the terms "comprise" and "include" are intended to specify the presence of stated features, integers, components or steps, but do not exclude the presence or addition of one or more other features, integers, components, steps or groups thereof.
The definitions of specific functional groups and chemical terms are described in more detail below. For the purposes of the present disclosure, chemical elements are identified according to the Periodic Table of the Elements, CAS version, Handbook of Chemistry and Physics, 75th edition, inside cover, and specific functional groups are generally defined as described therein. In addition, the general principles of organic chemistry and specific functional moieties and reactivity are described in Organic Chemistry, Thomas Sorrell, University Science Books, Sausalito, 1999; Smith and March, March's Advanced Organic Chemistry, 5th edition, John Wiley & Sons, Inc., New York, 2001; Larock, Comprehensive Organic Transformations, VCH Publishers, Inc., New York, 1989; Carruthers, Some Modem Methods of Organic

Synthesis, 3rd edition, Cambridge University Press, Cambridge, 1987.

All ranges cited herein are inclusive unless otherwise expressly indicated.

When a range of values is listed, it is intended to encompass every value and subrange within that range. For example, "$C_{1-6}$" is intended to encompass $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$ and $C_{5-6}$.

When any variable occurs more than once in any constituent or in Formula (I) or in any other formula depicting and describing compounds of the present disclosure, its definition at each occurrence is independent of its definition at every other occurrence. Furthermore, combinations of substituents and/or variables are permissible only if such combinations result in stable compounds.

As used herein, the term "deuterated" refers to one or more hydrogen atoms on a compound or group being substituted with deuterium. When a compound or group is deuterated, one, two, three or even more hydrogen atoms on the compound or group may be substituted with deuterium, up to the substitution of all hydrogen atoms on the compound or group with deuterium, at which point it may be referred to as a "perdeuterated compound or group".

In some embodiments, at the deuterated positions, the deuterium isotope abundance is greater than the natural deuterium isotope abundance (0.015%), preferably greater than 50%, more preferably greater than 60%, greater than 70%, greater than 80%, greater than 90%, greater than 95%, greater than 96%, greater than 97%, greater than 98%, greater than 99%, greater than 99.5%, or 100%.

In some cases, for example, when "hydrogen" and "deuterium" appear in a statement as parallel alternatives or when the description states that "hydrogen" is substituted with "deuterium", the term "hydrogen" represents the hydrogen isotope "$^1$hydrogen ($^1$H)", and the term "deuterium" represents the hydrogen isotope "$^2$hydrogen ($^2$H)"; or it is understood that at the specified position in the compound, the state in which hydrogen exists with the natural abundance of its isotopes is replaced by a state in which deuterium exists at an abundance greater than natural deuterium isotopic abundance (for example, deuterium abundance of greater than 50%, greater than 60%, greater than 70%, greater than 80%, greater than 90%, greater than 95%, greater than 96%, greater than 97%, greater than 98%, greater than 99%, greater than 99.5%, or 100%).

In some embodiments, when

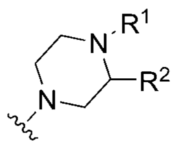

is optionally substituted with one deuterium atom, the deuterium atom may substitute on any ring carbon atom. In some embodiments, when

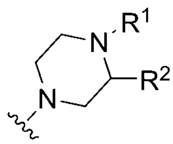

is optionally substituted with two or more deuterium atoms, these two or more deuterium atoms may substitute on the same ring carbon atom or on different ring carbon atoms.

In some embodiments, when $R^2$ is hydrogen, the

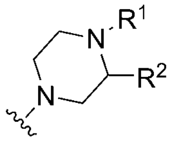 is 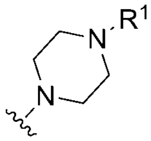 .

wherein hydrogens on the ring carbon atoms including $R^2$ may be substituted with 1 to 8 deuterium atoms, for example 1, 2, 3, 4, 5, 6, 7, or 8 deuterium atoms. These deuterium atoms may substitute on the same or different ring carbon atoms.

In some embodiments, when $R^1$ is non-deuterated methyl and $R^2$ is hydrogen, the

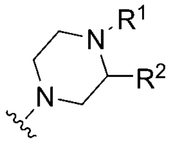 is 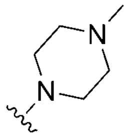 ,

wherein at least one hydrogen on the ring carbon atoms including $R^2$ is substituted with deuterium, for example, substituted with 1, 2, 3, 4, 5, 6, 7 or 8 deuterium atoms. These deuterium atoms may substitute on the same or different ring carbon atoms.

In preferred embodiments, optionally deuterated

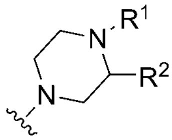

is selected from

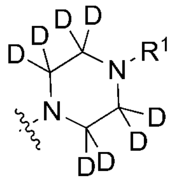 ,

$R^1$ is optionally non-deuterated, such as non-deuterated methyl, or deuterated, such as perdeuterated methyl, for example

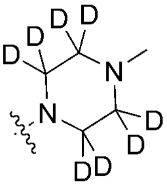 .

In some embodiments, when

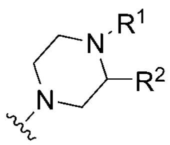 is 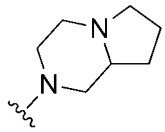

or its stereoisomers

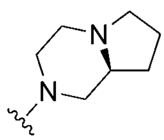 , 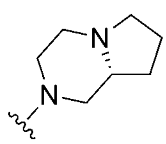

one or more deuterium atoms may substitute on any ring carbon atom; when there are two or more deuterium atoms, these two or more deuterium atoms may substitute on the same ring carbon atom, or may substitute on different ring carbon atoms. In some embodiments, when there are two or more deuterium atoms, these two or more deuterium atoms may substitute on one ring of a fused ring structure, or may substitute on two rings of a fused ring structure. In some embodiments, one or more deuterium atoms may substitute on a carbon atom common to two rings in a fused ring structure.

In a preferred embodiment, the optionally deuterated

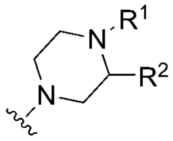

is selected from

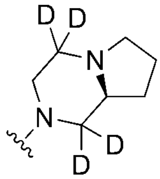 or 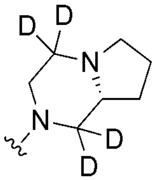 .

In some embodiments, when

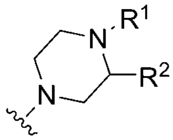

is non-deuterated

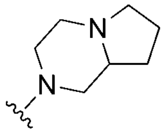 , 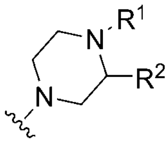

is

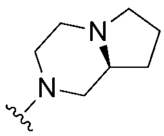 or 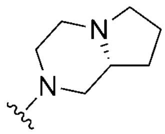 .

As used herein, the term "alkyl" refers to a straight or branched saturated hydrocarbon group. The term "$C_{i-j}$ alkyl" refers to an alkyl group having i to j carbon atoms. Unless otherwise indicated, an alkyl group may contain 1 to 10 carbon atoms. In certain embodiments, the alkyl group contains 1 to 6 carbon atoms, such as 1 to 5 carbon atoms, 1 to 4 carbon atoms, 1 to 3 carbon atoms, or 1 to 2 carbon atoms. Non-limiting examples of alkyl groups include methyl, ethyl, n-propyl and isopropyl, n-butyl, sec-butyl, isobutyl and tert-butyl, neopentyl, etc.

As used herein, the term "deuterated alkyl" refers to a substituent obtained by replacing one or more hydrogens on an alkyl group with deuterium. When an alkyl group is deuterated, one, two, three or even more hydrogen atoms on the alkyl group may be substituted with deuterium, up to the substitution of all hydrogen atoms on the alkyl group with deuterium, at which point it may be referred to as a "perdeuterated alkyl group". In some embodiments, non-limiting examples of deuterated alkyl groups include deuterated methyl groups, such as monodeuterated methyl, dideuterated methyl, trideuterated methyl (perdeuterated methyl), monodeuterated ethyl, dideuterated ethyl, trideuterated ethyl, tetradeuterated ethyl, pentadeuterated ethyl (perdeuterated ethyl) etc.

As used herein, the term "cycloalkyl" refers to non-aromatic, saturated monocyclic and polycyclic ring systems, wherein all ring-forming atoms are carbon. Unless otherwise indicated, a cycloalkyl group may contain 3 to 10 ring carbon atoms (i.e., $C_{3-10}$ cycloalkyl). In certain embodiments, the cycloalkyl group can include 3 to 9, 3 to 8, 3 to 7, 3 to 6, 4 to 10, 4 to 9, 4 to 8, 4 to 7, 4 to 6, 4 to 5, 5 to 10, 5 to 9, 5 to 8, 5 to 7, 5 to 6 ring carbon atoms, etc. In particular, cycloalkyl may be monocyclic or bicyclic. Alternatively, bicyclic cycloalkyl groups can include fused, spiro and bridged cycloalkyl structures.

In another aspect, also included are cycloalkyl rings wherein 1, 2, or 3 ring carbon atoms are replaced with heteroatoms. Such groups are referred to as "heterocyclyl" or "heterocycle", which refers to a cycloalkyl group as defined above but having at least one heteroatom selected from N, O, and S as ring-forming atoms. Unless otherwise indicated, a

heterocyclyl group may contain 3 to 10 ring-forming atoms (i.e. 3- to 10-menbered heterocyclyl). In certain embodiments, a heterocyclyl group may contain 3 to 9, 3 to 8, 3 to 7, 3 to 6, 4 to 10, 4 to 9, 4 to 8, 4 to 7, 4 to 6, 4 to 5, 5 to 10, 5 to 9, 5 to 8, 5 to 7, 5 to 6 ring-forming atoms etc. Especially, the heterocyclyl group can be monocyclic or bicyclic. Optionally, bicyclic heterocyclyl groups may include fused, spiro, and bridged heterocyclyl structures. Non-limiting examples of heterocyclyl groups include oxiranyl, pyrrolidinyl, piperidinyl, tetrahydropyranyl, piperazinyl, pyrrolidinyl, and morpholinyl. Heterocyclyl groups may also be described by the number of carbon atoms. For example, $C_{3-6}$ heterocyclyl refers to a heterocyclyl group containing three to six ring-forming carbon atoms, and may further contain at least one heteroatom, such as 1, 2 or 3 heteroatoms as ring-forming atoms. In certain embodiments, the heterocyclyl groups or heterocycles contain 1 or 2 heteroatoms as ring-forming atoms. In certain embodiments, the heterocyclyl groups can be monocyclic or bicyclic, such as fused bicyclic and spiro bicyclic. In the context of the present disclosure, the terms "heterocyclyl" and "heterocycle" can be used interchangeably.

As used herein, the term "deuterated heterocyclyl" refers to a substituent obtained by substituting one or more hydrogen atoms on a heterocyclyl with deuterium. When a heterocyclyl is deuterated, one, two, three or even more hydrogen atoms on the heterocyclyl may be substituted with deuterium, up to the substitution of all hydrogen atoms on the heterocyclyl group with deuterium, at which point it may be referred to as a "perdeuterated heterocyclyl group".

As used herein, the term "halo" or "halogen" refers to fluoride, chloride, bromide and iodide. In certain embodiments, non-limiting examples of halo include fluoride, chloride and bromide, more particularly fluoride and chloride.

As used herein, the term "heteroatom" refers to nitrogen (N), oxygen (O), and sulfur (S), and may include any oxidized forms of nitrogen and sulfur, and any quaternized forms of basic nitrogen, unless otherwise specified.

As used herein, the term "substituted" when referring to a chemical group means that the chemical group has one or more hydrogen atoms that are removed and replaced with a substituent. As used herein, the term "substituent" has its ordinary meaning known in the art and refers to a chemical moiety covalently attached to a parent group or, where appropriate, fused to the parent group. It is understood that the substitution of a given atom is limited by valence. It should be understood that substituents may be further substituted.

When it is indicated in Formula (I) or any embodiment thereof that a moiety is "optionally" substituted, this means that Formula (I) or its embodiments encompasses compounds wherein the moiety is substituted with the indicated substituent(s), and compounds wherein the moiety does not contain the indicated substituent(s) (i.e., wherein the moiety is unsubstituted)..

The compounds provided herein are described with reference to generic formulae and specific compounds. In addition, the compounds of the present disclosure may exist in a variety of different forms or derivatives, all of which are within the scope of the present disclosure. These include, for example, pharmaceutically acceptable salts, tautomers, stereoisomers, racemic mixtures, positional isomers, prodrugs, solvated forms, different crystalline forms or polymorphs, and active metabolites, etc.

As used herein, unless otherwise indicated, the term "pharmaceutically acceptable salt" includes salts that maintain the biological effectiveness of the free acid/base form of the specific compound and are not undesirable biologically or otherwise. Pharmaceutically acceptable salts may include salts formed with inorganic bases or acids and organic bases or acids. In the case where the compounds of the present disclosure contain one or more acidic or basic groups, the disclosure also includes their corresponding pharmaceutically acceptable salts. Therefore, compounds of the present disclosure containing acidic groups (e.g., carboxyl groups) may exist in salt form and may be used according to the disclosure as, for example, as alkali metal salts, alkaline earth metal salts, aluminum salts or ammonium salts. Further non-limiting examples of such salts include lithium salts, sodium salts, potassium salts, calcium salts, magnesium salts, barium salts or salts with ammonia or organic amines (e.g., ethylamine, ethanolamine, diethanolamine, triethanolamine, piperidine, N-methylglutamine, or amino acids). For example, these salts are readily obtained by reacting compounds having acidic groups with suitable bases (e.g., lithium hydroxide, sodium hydroxide, sodium propoxide, potassium hydroxide, potassium ethoxide, magnesium hydroxide, calcium hydroxide, or barium hydroxide). Other basic salts of compounds of the present disclosure include, but are not limited to, copper (I), copper (II), iron (II), iron (III), manganese (II), and zinc salts. Compounds of the present disclosure contain one or more basic groups, such as groups that can be protonated, they may exist in the form of salts, and may be used in the form of addition salts thereof with inorganic or organic acids according to the present disclosure. Examples of suitable acids include hydrochloric acid, hydrobromic acid, hydroiodic acid, phosphoric acid, sulfuric acid, nitric acid, methanesulfonic acid, p-toluenesulfonic acid, naphthalenedisulfonic acid, sulfoacetic acid, trifluoroacetic acid, oxalic acid, acetic acid, tartaric acid, lactic acid, salicylic acid, benzoic acid, carbonic acid, formic acid, propionic acid, pivalic acid, diethylacetic acid, malonic acid, succinic acid, pimelic acid, fumaric acid, malonic acid, maleic acid, malic acid, pamoic acid, mandelic acid, sulfamic acid, phenylpropionic acid, gluconic acid, ascorbic acid, isonicotinic acid, citric acid, adipic acid, taurocholic acid, glutaric acid, stearic acid, glutamic acid or aspartic acid, as well as other acids known to those skilled in the art. The salts formed are particularly hydrochlorides, chlorides, hydrobromides, bromides, iodides, sulfates, phosphates, methanesulfonates (mesylates), tosylates, carbonates, bicarbonates, formates, acetates, sulfoacetates, triflates, oxalates, malonates, maleates, succinates, tartrates, malates, pamoates, mandelates, fumarates, lactates, citrates, glutarates, stearates, aspartates and glutamates. Furthermore, the stoichiometry of salts formed from the compounds of the present disclosure may be integer or non-integer multiples of 1.

Compounds of the present disclosure containing basic nitrogen-containing groups can be quaternized using agents such as $C_{1-4}$ alkyl halides, for example, methyl, ethyl, isopropyl and tert-butyl chlorides, bromines and iodines; di-$C_{1-4}$ alkyl sulfates, for example, dimethyl, diethyl and diamyl sulfates; $C_{10-18}$ alkyl halides, for example, decyl, dodecyl, lauryl, myristyl and stearyl chlorides, bromides and iodides; and aryl $C_{1-4}$ alkyl halides, for example, benzyl chloride and phenethyl bromide.

If the compounds of the present disclosure contain both acidic and basic groups in the molecule, the present disclosure also includes inner salts or betaines (zwitterions) in addition to the above-mentioned salt forms. The corresponding salts may be obtained by conventional methods known to those skilled in the art, for example, by contacting them with organic or inorganic acids or bases in solvents or dispersants, or via anion exchange or cation exchange with other salts. The present disclosure further includes all salts of the compounds of the present disclosure, which are not directly suitable for use as pharmaceuticals due to low physiological compatibility, but can be used, for example, as intermediates for chemical reactions or for the preparation of pharmaceutically acceptable salts. For a review of more suitable salts, see Stahl and Wermuth, Handbook of Pharmaceutical Salts: Properties, Selection and Use (Wiley-VCH, 2002).

The compound of Formula (I) and its pharmaceutically acceptable salt may exist in both unsolvated and solvated forms. As used herein, the term "solvate" refers to a molecular complex comprising a stereoisomer, deuterated derivative or stereoisomeric deuterated derivative of a compound of Formula (I) or a pharmaceutically acceptable salt thereof, and one or more pharmaceutically acceptable solvent molecules. For example, the term "hydrate" is used when the solvent is water.

The compound of Formula (I) may have one or more chiral (asymmetric) centers. The present disclosure encompasses all stereoisomeric forms of compounds of Formula (I). The asymmetric centers present in the compound of Formula (I) may each independently have (R) or (S) configuration. When bonds to chiral carbons are depicted as straight lines in the structural formulae of the present disclosure, or when compound names are described without (R) or (S) chiral designations for chiral carbons, it is understood that both (R) and (S) configurations of each such chiral carbon and therefore each enantiomer or diastereomer and mixtures thereof are encompassed by said formula or name. The production of specific stereoisomers or mixtures thereof may be identified in examples where such stereoisomers or mixtures are obtained, but this in no way limits the inclusion of all stereoisomers and mixtures thereof within the scope of the present disclosure. When bonds to chiral carbons are depicted as solid or dashed wedges in the structural formulae of the present disclosure, or when compound names are described with (*R*) or (*S*) chiral designations for chiral carbons, it should be understood that the compound represented by the structural formula or name at this time has a definite stereoconfiguration at the chiral carbon position and will be distinguished from other stereoisomers or enantiomers or diastereomers or mixtures thereof.

The present disclosure includes all possible enantiomers and diastereomers as well as mixtures of two or more stereoisomers, for example mixtures of enantiomers and/or diastereomers in all ratios. Therefore, the enantiomers in the subject matter of the present disclosure are in enantiomerically pure forms (as levorotatory and dextrorotatory enantiomers), in racemic forms, and as mixtures of the two enantiomers in all proportions. In the case of cis/trans isomers, the present disclosure includes the cis form and trans form, as well as mixtures of these forms in all proportions. In the case of cis/trans isomers, the present disclosure includes the cis form and trans form, as well as mixtures of these forms in all proportions. If required, individual stereoisomers may be prepared by conventional methods (for example, through chromatography or crystallization, by using stereochemically homogeneous starting materials for synthesis, or via stereoselective synthesis) to separate the mixtures. Optionally, derivatization may be performed prior to separation of the stereoisomers. The separation of stereoisomer mixtures may be carried out in an intermediate step during the synthesis of the compound of Formula (I), or may be carried out on the final racemic product. Absolute stereochemistry may be determined by X-ray crystallography of crystalline products or crystalline intermediates, if necessary by derivatizing these crystalline products or intermediates with reagents containing stereocenters of known configuration. Alternatively, absolute stereochemistry may be determined by vibrational circular dichroism (VCD) spectroscopy analysis.

Unless otherwise indicated, structures described herein are also intended to include compounds that differ only in the presence of one or more isotopically enriched atoms, in other words, compounds in which one or more atoms are replaced by atoms with the same atomic number, but the atomic mass or mass number is different from the atomic mass or mass number that prevails in nature. Such compounds are referred to as "isotopic variants". The present disclosure is intended to include all pharmaceutically acceptable isotopic variants of compounds of Formula (I). Examples of isotopes suitable for inclusion in the compounds of the present disclosure include, but are not limited to, isotopes of hydrogen, such as $^{2}H$ (i.e., D, deuterium) and $^{3}H$ (i.e., tritium); carbon, such as $^{11}C$, $^{13}C$, and $^{14}C$; chlorine, such as $^{36}Cl$; fluorine, such as $^{18}F$; iodine, such as $^{123}I$ and $^{125}I$; nitrogen, such as $^{13}N$ and $^{15}N$; oxygen, such as $^{15}O$, $^{17}O$, and $^{18}O$; phosphorus, such as $^{32}P$; and sulfur, such as $^{35}S$. Certain isotopic variants of compounds of Formula (I), such as those incorporating radioactive

isotopes, can be used for drug and/or substrate tissue distribution studies. In particular, compounds having depicted structures that differ only in substitution with heavier isotopes (e.g., substitution of hydrogen with deuterium ($^2H$ or D)) can provide certain therapeutic advantages, for example, due to greater metabolic stability, increased *in vivo* half-life or reduced dosage requirements, and therefore can be used in some specific cases. Isotopic variants of compounds of Formula (I) can generally be prepared by conventional techniques known to those skilled in the art or by methods analogous to those described in the accompanying Examples, using appropriate isotopically labeled reagents in place of the non-labeled reagents previously employed for synthesis.

When describing the structure of a compound herein, for hydrogen atoms not designated as D (i.e., $^2H$) in the structural formula shown, it shall be generally understood that the hydrogen at this position exists either in the form of the hydrogen isotope "$^1$hydrogen ($^1H$)" or in a form with the natural isotopic abundance in the nature. For hydrogen atoms explicitly designated as D (i.e., $^2H$, deuterium) in the structure shown, it is understood that the hydrogen at this position exists either in the form of the hydrogen isotope "$^2$hydrogen ($^2H$, D, deuterium)" or in a form in which deuterium exists in a form with a deuterium isotopic abundance greater than the natural deuterium isotopic abundance (e.g., the deuterium abundance of greater than 50%, greater than 60%, greater than 70%, greater than 80%, greater than 90%, greater than 95%, greater than 96%, greater than 97%, greater than 98%, greater than 99%, greater than 99.5%, or 100%).

Pharmaceutically acceptable solvates according to the present disclosure may include those wherein the solvent of crystallization may be isotopically substituted, for example $D_2O$, $d_6$-acetone, $d_6$-DMSO.

One way to implement the present disclosure is to administer the compound of Formula (I) in the form of a prodrug. Therefore, certain derivatives of the compound of Formula (I) may have little or no pharmacological activity by themselves, and when administered in or on the body, for example through hydrolytic cleavage, particularly hydrolytic cleavage promoted by esterases or peptidases, are converted into the compound of Formula (I) with the desired activity. Such derivatives are referred to as "prodrugs". Further information on the use of prodrugs can be found in, for example, T. Higuchi and W. Stella, "Pro-drugs as Novel Delivery Systems", Vol. 14, ACS Symposium Series, and EB Roche (Ed.), "Bioreversible Carriers in Drug Design", Pergamon Press, 1987, American Pharmaceutical Association. Reference can also be made to Nature Reviews/Drug Discovery, 2008, 7, 355, and Current Opinion in Drug Discovery and Development, 2007, 10, 550.

Prodrugs according to the present disclosure can be prepared, for example, by replacing appropriate functional groups present in compounds of Formula (I) with certain moieties known to those skilled in the art, for example, the "promoieties" described in H. Bundgaard, "Design of Prodrugs", Elsevier, 1985, and Y. M. Choi-Sledeski and C. G. Wermuth, "Designing Prodrugs and Bioprecursors", Practice of Medicinal Chemistry, 4th edition, Chapter 28, 657-696, Elsevier, 2015. Thus, prodrugs according to the present disclosure can include, but are not limited to, (a) ester or amide derivatives of carboxylic acids in compounds of Formula (I), if any; (b) amide, imine, carbamate or amine derivatives of amino groups in compounds of Formula (I); (c) oxime or imine derivatives of carbonyl groups in compounds of Formula (I), if any; or (d) methyl, primary alcohol or aldehyde groups in compounds of Formula (I) which can be metabolically oxidized to carboxylic acids, if any.

References to the compounds of Formula (I) include the compounds themselves and prodrugs thereof. The present disclosure includes such compounds of Formula (I) as well as pharmaceutically acceptable salts of such compounds and pharmaceutically acceptable solvates of said compounds and salts.

## Use and Administration

The compounds of the present disclosure (stereoisomers, deuterated derivatives or stereoisomeric deuterated derivatives of the compounds of Formula (I)) - or pharmaceutically acceptable salts, solvates thereof, including mixtures thereof in all ratios - can be used as medicaments. They have been found to exhibit pharmacological activity that inhibits PLK1. It is hypothesized that through this activity, the compounds of the present disclosure may prevent or reverse dysregulation of PLK1 activity. By preventing such dysregulation of PLK1 activity, it is able to exert its effect as a tumor suppressor. In addition to preventing or reversing dysregulation of PLK1 activity, the pharmacological activity of the compounds of the present disclosure may also be used in other pathophysiological conditions where inhibition of PLK1 would be beneficial.

Therefore, the compounds of the present disclosure as PLK1 inhibitors are particularly useful for treating diseases and conditions caused by dysregulated PLK1 activity and/or diseases and conditions associated with PLK1, such as cancer. The diseases and conditions caused by dysregulated PLK1 activity and/or diseases and conditions associated with PLK1 include but not limited to cancers such as leukemia, lymphoma, pancreatic cancer, breast cancer, prostate cancer, lung cancer, ovarian cancer, colorectal cancer, liver cancer, gastric cancer, esophageal cancer, melanoma, multiple myeloma, and sepsis, etc. Without wishing to be bound to any particular theory or explanation, it may be hypothesized that these compounds may be able to achieve this objective through acting directly on cancer cells and/or indirectly by modulating the immune system’s response to tumors.

The compounds of the present disclosure may be administered in an amount effective to treat the diseases or

conditions described herein. The compounds of the present disclosure may be administered as the compounds themselves, or alternatively, as pharmaceutically acceptable salts. For administration and dosing purposes, the compounds of the present disclosure themselves (stereoisomers, deuterated derivatives or stereoisomeric deuterated derivatives of compounds of Formula (I)) or their pharmaceutically acceptable salts, solvates will be referred to collectively as the compounds of the present disclosure.

The compounds of the present disclosure are administered by any suitable route in the form of pharmaceutical compositions appropriate for such routes, and at doses effective for the intended treatment. The compounds of the present disclosure may be administered orally, rectally, vaginally, parenterally or topically.

As used herein, the term "administer" refers to absorbing, ingesting, injecting, inhaling, implanting or other means of introducing a compound of the present disclosure or pharmaceutical composition thereof. The term "treatment" refers to reversing, alleviating, delaying the onset of, or inhibiting the progression of "pathological conditions" (e.g., diseases, disorders or conditions, or one or more signs or symptoms thereof) described herein. In certain embodiments, treatment may be administered after one or more signs or symptoms of the disease or condition have developed or have been observed. In other embodiments, treatment may be administered in the absence of signs or symptoms of the disease or condition. For example, susceptible individuals may be treated prior to the onset of symptoms (e.g., according to a history of symptoms and/or according to genetic or other susceptibility factors). Treatment may also be continued after symptoms have resolved, such as to delay or prevent recurrence. As used herein, the terms "disease", "disorder", "condition", and "pathological condition" are used interchangeably.

Those skilled in the art can determine the dosage level of administration by routine experiments. The dosage regimen of the compounds of the present disclosure and/or compositions containing said compounds is based on multiple factors including: the type, age, weight, sex and medical condition of the patient; the severity of the condition; the route of administration; and the activity of the specific compound used. Therefore, the dosage regimen may vary widely. For example, the dosage level of the compounds of the present disclosure may range from about 0.001 to about 100 mg/kg (i.e., mg per kg of body weight) per day. In certain embodiments, the total daily dose of the compounds of the present disclosure administered in a single or divided dose may be about 0.001 to about 10 mg/kg. It is not uncommon that the administration of the compounds disclosed herein is repeated multiple times in one day.

Pharmaceutical Composition

In some aspects, the present disclosure relates to a pharmaceutical composition comprising the stereoisomer, deuterated derivative or stereoisomeric deuterated derivative of a compound of Formula (I) as provided herein, or a pharmaceutically acceptable salt, solvate thereof, and at least one pharmaceutically acceptable carrier or excipient.

As used herein, the term "pharmaceutically acceptable carrier or excipient" refers to a carrier or excipient that can be used to prepare a pharmaceutical composition, which is generally safe, non-toxic and not biologically or otherwise undesirable, and includes carriers or excipients that are acceptable for veterinary use as well as human pharmaceutical use. Pharmaceutically acceptable carriers or excipients as used herein include one and more than one such carrier or excipient. The specific carrier or excipient used will depend on the manner and purpose of applying the compounds of the present disclosure. Suitable carriers and excipients are well known to those skilled in the art and are described in detail in, for example, Ansel, Howard C et al., Ansel's Pharmaceutical Dosage Forms and Drug Delivery Systems. Philadelphia: Lippincott, Williams & Wilkins, 2004; Gennaro, Alfonso R. et al., Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. One or more of buffers, stabilizers, surfactants, wetting agents, lubricants, emulsifiers, suspending agents, preservatives, antioxidants, opacifiers, glidants, processing aids, colorants, sweeteners, aromatizing agent, flavorants, diluents and other known additives may also be included to provide a refined presentation of the drug (i.e., the compound or pharmaceutical composition provided herein) or to aid in the production of a pharmaceutical product (i.e., the medicament).

The compositions of the present disclosure can be formulated into a variety of forms. These include, for example, liquid, semisolid and solid dosage forms, such as liquid solutions (e.g., injectable and infusible solutions), dispersions or suspensions, tablets, pills, powders, liposomes, suppositories, etc. The form depends on the intended route of administration and therapeutic application.

The pharmaceutical compositions of the present disclosure can be prepared by any well-known pharmaceutical techniques (e.g., effective formulations and administration procedures). The above considerations on effective formulations and administration procedures are well known in the art and are described in standard textbooks. For example, the preparation of the pharmaceutical product is discussed in Hoover, John E., Remington's Pharmaceutical Sciences, Mack Publishing Co., Easton, Pennsylvania, 1975; Liberman et al., ed., Pharmaceutical Dosage Forms, Marcel Decker, New York, NY, 1980; and Kibbe et al., ed., Handbook of Pharmaceutical Excipients, 3rd edition, American Pharmaceutical Association, Washington, 1999.

In yet another aspect, the present disclosure relates to a kit for treating diseases and conditions caused by

dysregulated PLK1 activity and/or diseases and conditions associated with PLK1, comprising the stereoisomer, deuterated derivative or stereoisomeric deuterated derivative of a compound of Formula (I), or a pharmaceutically acceptable salt, solvate thereof as provided herein, or the pharmaceutical composition comprising the stereoisomer, deuterated derivative or stereoisomeric deuterated derivative of a compound of Formula (I), or a pharmaceutically acceptable salt, solvate thereof as provided herein, a container, and optionally a package insert or label indicating treatment.

Treatment Methods

In yet another aspect, the present disclosure relates to a method for treating diseases and conditions caused by dysregulated PLK1 activity and/or diseases and conditions associated with PLK1 in a subject in need thereof, comprising administering to the subject a therapeutically effective amount of the stereoisomer, deuterated derivative or stereoisomeric deuterated derivative of a compound of Formula (I), or a pharmaceutically acceptable salt, solvate thereof as provided herein, due to the PLK1 inhibitory activity of the compounds of the present disclosure.

As used herein, the term "subject in need thereof" is a subject suffering from a disease and condition caused by dysregulated PLK1 activity and/or a disease and condition associated with PLK1, or a subject having an increased risk of developing a PLK1-related disease or condition relative to the population as a whole. In certain embodiments, the subject is a warm-blooded animal. In certain embodiments, the warm-blooded animal is a mammal. In certain embodiments, the warm-blooded animal is a human.

As used herein, the term "diseases and conditions caused by dysregulated PLK1 activity and/or diseases and conditions associated with PLK1" refers to any pathophysiological condition in which inhibition of PLK1 would be beneficial. In certain embodiments, the diseases and conditions caused by dysregulated PLK1 activity and/or diseases and conditions associated with PLK1 are cancers. In certain embodiments, the diseases and conditions caused by dysregulated PLK1 activity and/or diseases and conditions associated with PLK1 are diseases and conditions selected from the group consisting of leukemia, lymphoma, pancreatic cancer, breast cancer, prostate cancer, lung cancer, ovarian cancer, colorectal cancer, liver cancer, gastric cancer, esophageal cancer, melanoma, multiple myeloma, sepsis, and the like.

In another aspect, the present disclosure relates to the stereoisomer, deuterated derivative or stereoisomeric deuterated derivative of a compound of Formula (I), or a pharmaceutically acceptable salt, solvate thereof as provided herein for use in treating diseases and conditions caused by dysregulated PLK1 activity and/or diseases and conditions associated with PLK1.

In yet another aspect, the present disclosure relates to the use of the stereoisomer, deuterated derivative or stereoisomeric deuterated derivative of a compound of Formula (I), or a pharmaceutically acceptable salt, solvate thereof as provided herein, in the preparation of medicaments for treating diseases and conditions caused by dysregulated PLK1 activity and/or diseases and conditions associated with PLK1.

Synthesis

The compounds of the present disclosure can be prepared by the general and specific methods described below using the common knowledge of those skilled in the art of synthetic organic chemistry. Such common knowledge can be found in standard reference books, for example, Barton and Ollis (eds.), Comprehensive Organic Chemistry, Elsevier; Richard Larock, Comprehensive Organic Transformations: A Guide to Functional Group Preparation, John Wiley and Sons; and Compendium of Organic Synthesis Methods, Volumes I-XII, Wiley-Interscience.

The schemes described below are intended to provide a general description of methods for preparing compounds of the present disclosure. Some compounds of the present disclosure may contain single or multiple chiral centers with stereochemical designations (R) or (S). It will be apparent to those skilled in the art that all synthetic transformations can be performed in a similar manner, regardless of whether the material is enantiomerically enriched or racemic. In addition, the resolution of the desired optically active material can be performed at any desired point in the procedure using well-known methods, such as those described herein and in the chemical literature.

## Examples

To describe the present disclosure in more detail, the following examples are provided. The examples described herein are intended to illustrate the compounds, methods, and compositions provided herein, and should not be construed in any way as limiting their scope.

During the synthesis process, it may be necessary and/or desirable to protect sensitive or reactive groups on any of the molecules involved. This can be achieved through conventional protecting groups, such as those described in T.W. Greene and P.G.M. Wuts, Protective Groups in Organic Synthesis, 4th Edition, John Wiley & Sons. The protective groups are optionally removed at a convenient subsequent stage using methods well known in the art.

The compounds of the present disclosure can be readily prepared according to the following reaction schemes and examples or modifications thereof, using readily available starting materials, reagents and conventional synthesis procedures. In these reactions, variants known to those skilled in the art but not mentioned in more detail may also be used. In addition, according to the reaction schemes and examples described herein, other methods for preparing the compounds of the present disclosure will be apparent to those skilled in the art. Unless otherwise indicated, all variables are defined as above. In general, in chemical procedures, all reagents and starting materials can be purchased from commercial suppliers or can be easily prepared by those skilled in the art.

Exemplary compounds are shown in Table 1.

**Table 1**

| Compound ID | Structure |
|---|---|
| **CPD01** | 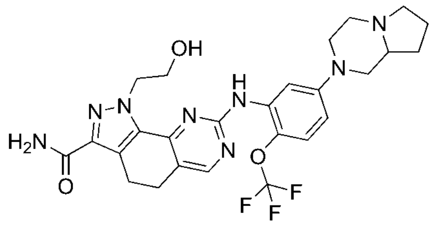 |
| **CPD02** | 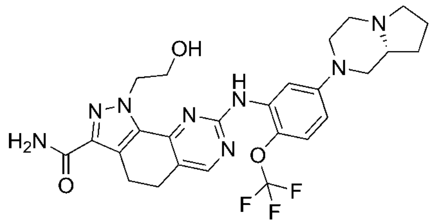 |
| **CPD03** | 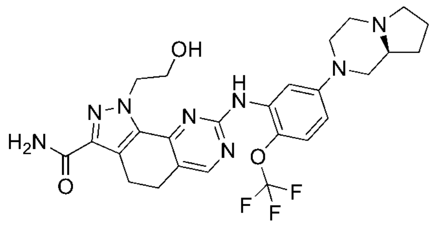 |
| **CPD04** | 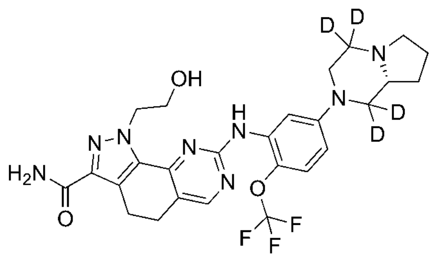 |
| **CPD05** | 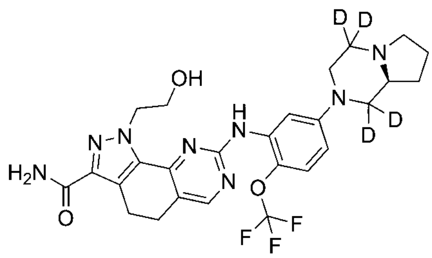 |

(continued)

| Compound ID | Structure |
|---|---|
| CPD06 | 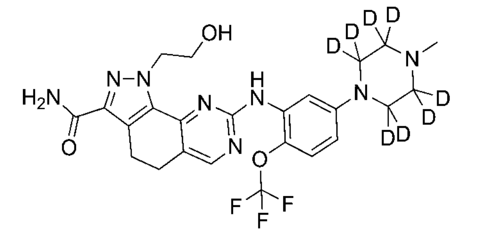 |
| CPD07 | 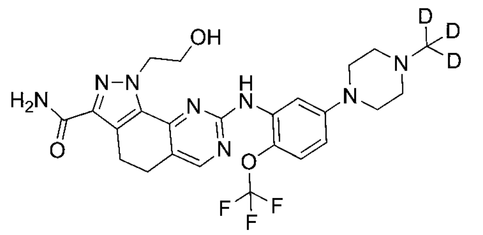 |

**Preparation of Intermediates**

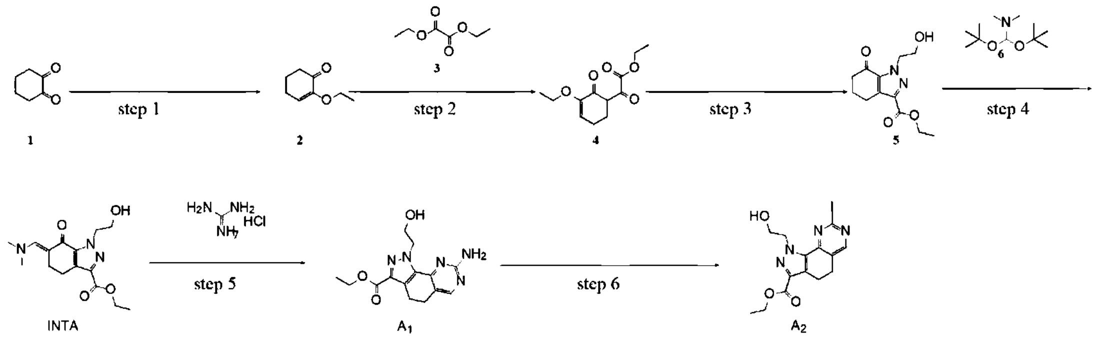


Step 1. Synthesis of Compound 2

At room temperature (RT), Compound 1 (40.0 g, 408.2 mmol, 1.0 eq) was dissolved in EtOH (40 mL), p-toluenesulfonic acid PTSA (7.0 g, 40.8 mmol, 0.1 eq) was added, and the mixture was stirred and refluxed overnight. After TLC showed that no Compound 1 remained, the mixture was concentrated. The residue was dissolved in EtOAc (200 mL), washed with saturated sodium bicarbonate solution (100 mL × 3), dried over anhydrous $Na_2SO_4$, and concentrated to give a crude product. The residue was purified by silica gel column chromatography (petroleum ether/EtOAc = 100:1) to obtain Compound 2 (30.0 g, 60.1%) as a yellow oil. $^1$HNMR (300 MHz, DMSO-$d_6$): $\delta$ ppm 5.98 (t, $J$ = 4.5 Hz, 1H), 3.67 (q, $J$ = 6.9 Hz, 2H), 2.46 - 2.24 (m, 4H), 1.95 - 1.77 (m, 2H), 1.22 (t, $J$ = 6.9 Hz, 3H). LCMS: 141.1 ([M+H]$^+$).

Step 2. Synthesis of Compound 4

Compound 2 (20.0 g, 142.7 mmol, 1.0 eq) was dissolved in THF (250 mL), and LiHMDS (171 mL, 171.2 mmol, 1.2 eq, 1 M in THF) was added dropwise at -50°C under nitrogen protection. After the addition was completed, stirring was continued at this temperature for 30 minutes. Compound 3 (22.2 mL, 157.0 mmol, 1.1 eq) was added dropwise at -50°C. After the addition was completed, the mixture was allowed to warm to room temperature and stirred overnight. After LC-MS showed complete consumption of Compound 2, 1 N HCl (about 180 mL) was used to adjust the pH to 4 under an ice-water bath. The mixture was extracted with ethyl acetate (300 mL × 3). The organic phases were combined and dried over anhydrous sodium sulfate. The solvent was removed by rotary evaporation to obtain a residue, which was further purified by column chromatography to obtain Compound 4 (20.0 g, 58.3%) as a yellow oil. $^1$H NMR (300 MHz, $CDCl_3$): $\delta$ ppm 14.93 (s, 1H), 5.88 (d, $J$ = 4.8 Hz, 1H), 4.34 (q, $J$ = 6.9 Hz, 2H), 3.82 (q, $J$ = 6.9 Hz, 2H), 2.89 (t, $J$ = 6.9 Hz, 2H), 2.40 (q, $J$ = 5.1 Hz, 2H), 1.50 - 1.32 (m, 6H).

Step 3. Synthesis of Compound 5

Compound 4 (20.0 g, 83.3 mmol, 1.0 eq) was dissolved in acetic acid (200 mL), and 2-hydroxyethylhydrazine (6.3 g, 83.3 mmol, 1.0 eq) was added under stirring at room temperature. The mixture was stirred for 2 hours at room temperature until LCMS monitoring showed complete consumption of Compound 4. The reaction solution was concentrated, and the residue was dissolved in 500 mL of ethyl acetate, washed with water, saturated sodium bicarbonate three times, and saturated brine three times. After drying over anhydrous sodium sulfate, the desiccant was filtered out, and the solvent was removed by rotary evaporation to obtain a light yellow Compound 5 (16.0 g, 76.2%). $^1$H NMR (300 MHz, DMSO-$d_6$): $\delta$ ppm 4.86 (t, $J$ = 5.7 Hz, 1H), 4.54 (t, $J$ = 5.7 Hz, 2H), 4.29 (q, $J$ = 6.9 Hz, 2H), 3.72 (q, $J$ = 5.7 Hz, 2H), 2.93 (t, $J$ = 6.0 Hz, 2H), 2.62 - 2.42 (m, 2H), 2.13 - 1.95 (m, 2H), 1.30 (t, $J$ = 7.2 Hz, 3H). LCMS: 253.1 ([M+H]$^+$).

Step 4. Synthesis of Intermediate Int-A

Compound 5 (20.0 g, 79.3 mmol, 1.0 eq) was dissolved in DMF (180 mL) and Compound 6 (48.3 g, 237.9 mmol, 3.0 eq) was added, and the mixture was stirred at 60°C for 2 hours. After LCMS monitoring showed complete consumption of Compound 5, the mixture was allowed to cool to room temperature, the reaction solution was poured into 1000 mL of water, extracted with ethyl acetate 6 times, the organic phases were combined, washed with saturated brine, dried over anhydrous sodium sulfate, the desiccant was filtered out, and the solvent was removed by rotary evaporation to obtain a light yellow Intermediate Int-A (13.0 g, 66.7%) without further purification. $^1$H NMR (300 MHz, DMSO-$d_6$): $\delta$ ppm 7.48 (s, 1H), 4.84 (brs, 1H), 4.60 (t, $J$ = 6.0 Hz, 2H), 4.27 (q, $J$ = 7.2 Hz, 2H), 3.78 - 3.65 (m, 2H), 3.11 (s, 6H), 2.96 - 2.86 (m, 2H), 2.86 - 2.76 (m, 2H), 1.29 (t, $J$ = 7.2 Hz, 3H).

Step 5. Synthesis of Intermediate A1

The Intermediate Int-A (6.0 g, 0.7 mmol, 1.0 eq) was dissolved in DMF (60 mL), and Compound 7 (4.7 g, 48.8 mmol, 2.5 eq) was added. The mixture was reacted at 110°C for 12 hours under $N_2$ protection. The reaction was monitored by LCMS until no INT-A remained. The mixture was allowed to cool to room temperature, the reaction solution was poured into 180 mL of water, extracted with ethyl acetate three times, the organic phases were combined, washed with saturated brine, dried over anhydrous sodium sulfate, the desiccant was filtered out, and the solvent was removed by rotary evaporation to obtain a crude product, which was purified by column chromatography to obtain a light yellow intermediate (3.6g, 60.8%). $^1$H NMR (400 MHz, $CDCl_3$): $\delta$ ppm 8.20 (s, 1H), 5.10 (s, 2H), 4.93 (t, $J$ = 4.8 Hz, 2H), 4.42 (q, $J$ = 7.2 Hz, 2H), 4.13 (t, $J$ = 4.8 Hz, 2H), 3.06 (t, $J$ = 7.6 Hz, 2H), 2.81 (t, $J$ = 7.6 Hz, 2H), 1.40 (t, $J$ = 7.2 Hz, 3H). LCMS: 304.1 ([M+H]$^+$).

Step 6. Synthesis of Intermediate A2

Intermediate A1 (4.0 g, 13.2 mmol, 1.0 eq) was dissolved in DME (320 mL), and CsI (6.9 g, 26.4 mmol, 2.0 eq), $I_2$ (3.3 g, 13.2 mmol, 1.0 eq), CuI (1.0 g, 5.3 mmol, 0.4 eq) and isoamyl nitrite (4.6 g, 39.6 mmol, 3.0 eq) were added. The reaction solution was reacted at 80°C for 3 hours, allowed to cool to room temperature, and a white solid was precipitated. The white solid was filtered out and dissolved in 200 mL of dichloromethane. The solution was washed with aqueous ammonia, sodium thiosulfate solution, and saturated brine once, and dried over anhydrous sodium sulfate. The desiccant was filtered out, and the solvent was removed by rotary evaporation to obtain a crude product, which was purified by column chromatography to obtain a yellow Intermediate A2 (2.0 g, 36.6%). $^1$H NMR (400 MHz, $CDCl_3$): $\delta$ ppm 8.30 (s, 1H), 4.92 (t, $J$ = 4.8 Hz, 2H), 4.42 (q, $J$ = 7.2 Hz, 2H), 4.14 (t, $J$ = 4.8 Hz, 2H), 3.15 (t, $J$ = 7.2 Hz, 2H), 2.94 (t, $J$ = 8.0 Hz, 2H), 1.41 (t, $J$ = 7.2 Hz, 3H). LCMS: 415.1 ([M+H]$^+$).

**Synthesis of Intermediate A4**

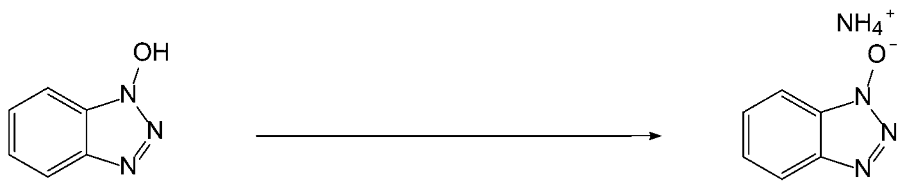

HOBT

$A_4$

HOBT (4.0 g, 29.6 mmol, 1.0 eq) was dissolved in methanol (40 mL), and aqueous ammonia (2.4 mL) was added and stirred at room temperature for 5 hours to precipitate a solid. The solid was filtered to obtain a white solid, which was washed with petroleum ether and dried to obtain Intermediate A4 (3.6 g, 79.9%).

**Synthesis of Intermediate A8**

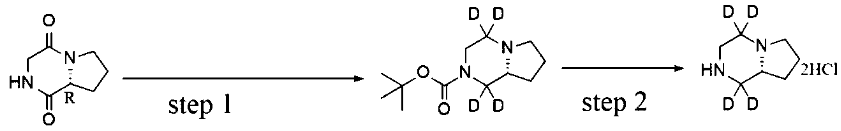


$A_6$ $A_7$ $A_8$

Step 1. Synthesis of A7 ((R)-tert-butyl hexahydropyrrolo[1,2-a]pyrazine-2(1H)-carboxylate-1,1,4,4-d4)

A6 (12 g, 77.4 mmol) was dissolved in a mixed solution of $CD_3OD$ (25 mL) and DCM (500 mL), heated to reflux for 8 h, and the solvent was removed by rotary evaporation. The residue was redissolved in $CD_3OD$ (25 mL) and DCM (500 mL), refluxed for 8 h, and the solvent was removed to obtain a white solid. The solid was dissolved in THF (200 mL), and $LiAlD_4$ (8.13 g, 193.5 mmol) was added at room temperature. The mixture was refluxed for 1 h, allowed to cool to 0°C, and DCM (500 mL), water (8.13 mL), and 15% NaOH (8.13 mL) were added. $(Boc)_2O$ (33.75 g, 154.9 mmol) and TEA (23.46 g, 232.2 mmol) were added to the reaction solution, stirred at room temperature for 8 hours, the insoluble matter was filtered off, the filtrate was concentrated, and purified by silica gel column chromatography to obtain A7 as a brown oil, crude product (12.7 g, 71%), LCMS: 231.2 $[M+H]^+$.

Step 2. Synthesis of A8

The Intermediate A7 was dissolved in MeOH (10 mL), and 4N HCl in dioxane (10 mL) was added to react at room temperature for 16 hours. The solvent was removed without further purification to obtain a crude product A8 (1.26 g).

**Synthesis Example 1. Synthesis of Compounds CPD02 and CPD03**

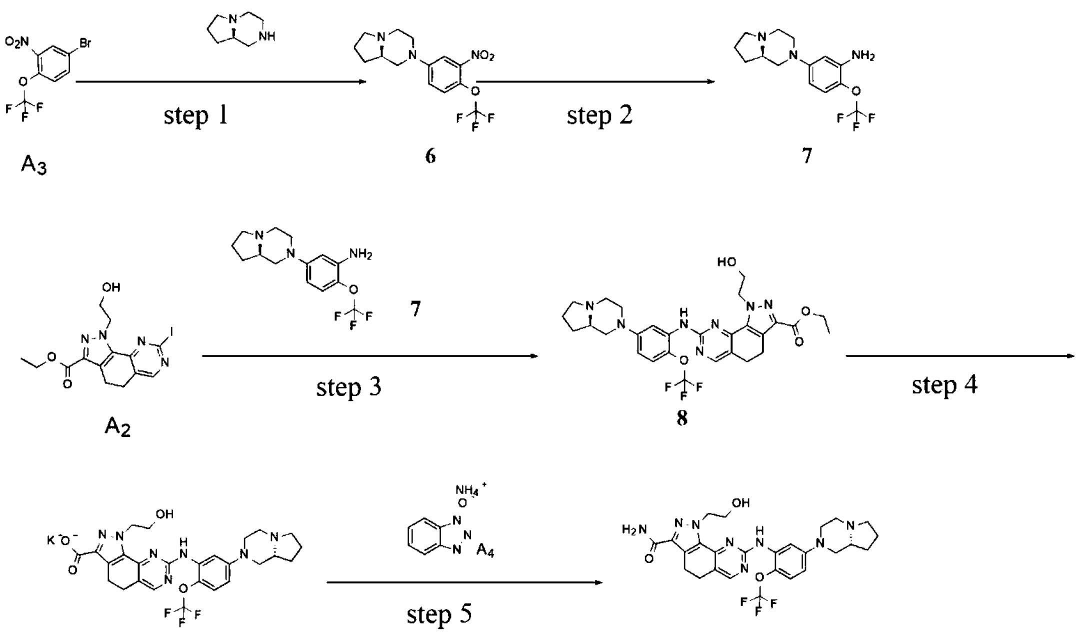


$A_3$ 6 7 $A_2$ 7 8 9 $A_4$ CPD 02

Step 1. Synthesis of Compound 6

1,4-Dioxane (55 mL) was added to a mixture of Intermediate A3 (550.0 mg, 1.9 mmol, 1.0 eq), X-phos (190.7 mg, 0.4 mmol, 0.2 eq), (R)-octahydropyrrolo[1,2-A]pyrazine (718 mg, 5.7 mmol, 3 eq) and $Cs_2CO_3$ (1.2 g, 3.8 mmol, 2.0 eq), and $Pd_2(dba)_3$ (183.1 mg, 0.2 mmol, 0.1 eq) was added at room temperature. The reaction system gas was purged with $N_2$ three times, and the reaction was heated to 90°C under $N_2$ protection for 6 hours. The reaction solution was allowed to cool to room temperature, concentrated, and 15 mL of water was added. The product was extracted with dichloromethane three times, and the organic phase was washed with saturated brine three times and dried over anhydrous sodium sulfate. The desiccant was filtered off, and the solvent was removed by rotary evaporation to obtain a crude product, which was purified by column chromatography to obtain Compound 6 (370 mg, 58.1%) as a light yellow solid, LCMS: 332.1 ($[M+H]^+$).

Step 2. Synthesis of Compound 7

Compound 6 (370.0 mg, 1.1 mmol, 1.0 eq) was dissolved in ethyl acetate (7.4 mL) and Pd/C (148.0 mg, 40% wt) was added. The mixture was reacted at room temperature overnight under $H_2$, the catalyst was filtered off, and the mixture was concentrated to give a crude product of Compound 7 (300 mg), which was used directly in subsequent reactions without purification. LCMS: 302.7 ($[M+H]^+$).

Step 3. Synthesis of Compound 8

In a glove box, DMF (2 mL) was added to a mixture of Intermediate A2 (120.0 mg, 0.29 mmol, 1.0 eq), Compound 7 (174.6 mg, 0.58 mmol, 2.0 eq), $K_2CO_3$ (120.1 mg, 0.87 mmol, 3.0 eq), BINAP (72.2 mg, 0.12 mmol, 0.4 eq) and $Pd(OAc)_2$ (9.7 mg, 0.06 mmol, 0.2 eq). After the addition, the mixture was reacted at 120°C under $N_2$ protection for 4 hours. After LCMS monitoring showed complete consumption of Intermediate A2, the reaction temperature was allowed to cool to room temperature, water (10 mL) was added, and the mixture was extracted with EtOAc three times. The organic phase was washed with saturated brine three times and dried over anhydrous sodium sulfate. The desiccant was filtered off, and the solvent was removed by rotary evaporation to obtain a crude product, which was purified by column chromatography to obtain Compound 8 (100 mg, 58.7%) as a brown oil. LCMS: 588.2 ($[M+H]^+$).

Step 4. Synthesis of Compound 9

Compound 8 (100 mg, 0.17 mmol, 1.0 eq) was dissolved in MeOH (1 mL), THF (1 mL) and $H_2O$ (1 mL), KOH (19.0 mg, 0.34 mmol, 2.0 eq) was added, and the mixture was stirred at room temperature for 2 hours. The reaction solution was concentrated, 5 mL of water was added to the residue, and the mixture was extracted with ethyl acetate three times. The aqueous phase was lyophilized to obtain a gray crude product of Compound 9 (80 mg). LCMS: 560.2 ($[M-K+H+H]^+$, signal of the acid form).

Step 5. Synthesis of Compound CPD02

The above Compound 9 (80 mg, 0.14 mmol, 1.0 eq) was dissolved in DMF (2 mL), and EDCI (80.2 mg, 0.42 mmol, 3.0 eq) and Intermediate A4 (63.8 mg, 0.42 mmol, 3.0 eq) were added. The mixture was stirred at room temperature for 4 hours. After HPLC monitoring showed complete consumption of Compound 9, 10 mL of water was added. The mixture was extracted with ethyl acetate three times. The organic phases were combined, and dried over anhydrous sodium sulfate. The desiccant was filtered out, and the solvent was removed by rotary evaporation to obtain a crude product. The crude product was purified by preparative HPLC (Prep-HPLC) (mobile phase: acetonitrile, 0.1% formic acid and water from 5% to 35%) and lyophilized to obtain a yellow solid powder CPD02 (10.0 mg, 2-step comprehensive yield 10.5%). $^1H$ NMR (400 MHz, DMSO-$d_6$): $\delta$ ppm 8.85 (s, 1H), 8.33 (s, 1H), 7.43 (s, 1H), 7.31-7.23 (m, 3H), 6.80 (dd, *J* = 9.2, 2.1 Hz, 1H), 4.62 (t, *J* = 5.2 Hz, 2H), 3.76 (d, *J* = 9.6 Hz, 1H), 3.70-3.58 (m, 3H), 3.10-2.90 (m, 4H), 2.85-2.69 (m, 3H), 2.42 (t, *J* = 10.4 Hz, 1H), 2.21 (td, *J* = 11.2, 3.2 Hz, 1H), 2.13-1.95 (m, 2H), 1.90-1.76 (m, 1H), 1.76-1.60 (m, 2H), 1.45-1.27 (m, 1H). LCMS: 559.1 ($[M+H]^+$).

The synthesis of Compound CPD03 is similar to that of CPD02. The structural spectrum is $^1H$ NMR (300 MHz, DMSO-$d_6$): $\delta$ ppm 8.86 (brs, 1H), 8.34 (s, 1H), 7.44 (s, 1H), 7.29-7.15 (m, 3H), 6.80 (dd, *J* = 9.1, 2.9 Hz, 1H), 4.66-4.54 (m, 3H), 3.80-3.70 (m, 1H), 3.68-3.54 (m, 3H), 3.08-2.93 (m, 4H), 2.83-2.69 (m, 3H), 2.31-2.22 (m, 1H), 2.22-2.14 (m, 1H), 2.09-1.96 (m, 2H), 1.86-1.64 (m, 3H), 1.43-1.29 (m, 1H). LCMS: 559.3 ($[M+H]^+$).

**Synthesis Example 2. Synthesis of Compound CPD04**

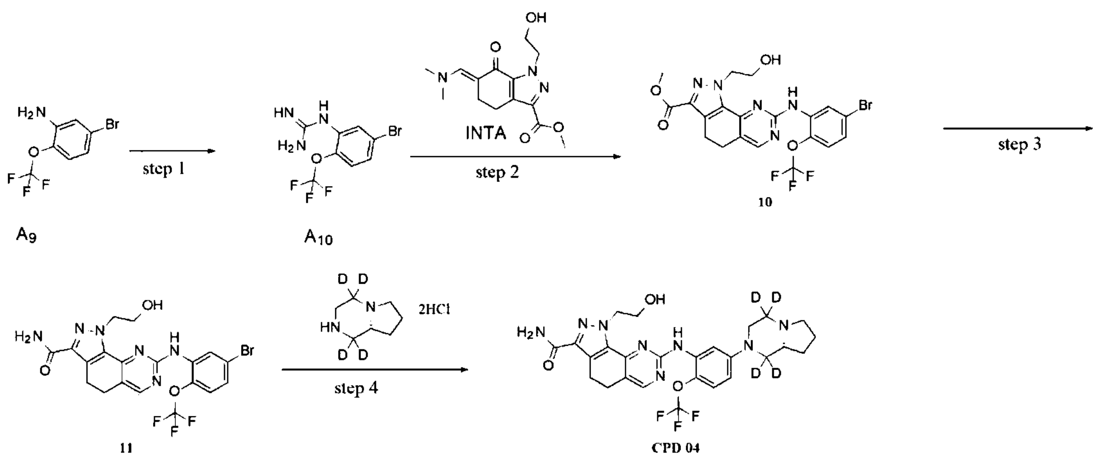


A9 A10 10 11 CPD 04

Step 1. Synthesis of A10

5-Bromo-2-trifluoromethoxyaniline A9 (10.0 g, 39.0 mmol) was dissolved in EtOH (30 mL) and added dropwise to a solution containing cyanamide (3.28 g, 78 mmol), EtOH (10 mL) and $H_2O$ (2 mL). A mixture of 37% HCl ( 6.5 mL) and EtOH (20 mL) was added dropwise to the mixture and refluxed for 5 days. The reaction solution was allowed to cool to room temperature, concentrated and diluted with water; 1N NaOH was added until the reaction solution became alkaline, extracted with ethyl acetate several times, dried over sodium sulfate and concentrated to obtain A10 (3.2 g, 27%).

Step 2. Synthesis of Intermediate 10

A10 (29.3 g, 98.3 mmol, 1.2 eq) was dissolved in DMF (400 mL), and a solution of the Intermediate INT-A(24 g, 81.9 mmol, 1.0 eq) in DMF (200 mL) was added. The reaction solution was reacted at 110°C for 16 hours, allowed to cool to room temperature, poured into ice water, and the insoluble matter was filtered to obtain Compound 10 (21.2 g, 50%) as a white solid. LCMS: 528.2, 530.2 ($[M+H]^+$).

Step 3. Synthesis of Intermediate Compound 11

Compound 10 (21.2 g, 40.1 mmol, 1.0 eq) was dissolved in THF (500 mL), and $NH_4Cl$ (6.44 g, 120 mmol, 3.0 eq) and LiHMDS (240 mmol, 6.0 eq) were added, and stirred at room temperature for 30 minutes. The solvent was removed by rotary evaporation, and the residue was washed with water and dried to obtain Compound 11 (16.8 g, 81.8%) as a white solid, LCMS: 513.1, 515.1 ($[M+H]^+$).

Step 4. Synthesis of CPD04

A mixture of Compound 11 (2.0 g, 3.9 mmol), 1.0 eq), Intermediate A8 (1.26 g, 6.25 mmol, 1.6 eq), $Pd_2(dba)_3$ (285 mg, 0.312 mmol, 0.08 eq) and Davephos (123 mg, 0.312 mmol, 0.08 eq) were added with THF (100 mL) and LiHMDS (40 mL, 40 mmol). The mixture was reacted at 100°C for 2 hours, and water was added to quench the reaction. The reaction solution was extracted with EtOAc (50 mL × 3). The organic phases were combined and the solvent was removed by rotary evaporation. The residue was purified by silica gel column chromatography to obtain the target product CPD04 (1 g, 45.6%) as a white solid. LCMS: 563.3 $[M+H]^+$. $^1H$ NMR (400 MHz, Methanol-$d_4$) $\delta$ 8.30 (s, 1H), 7.57 (d, 1H), 7.26 (dd, 1H), 6.85 (dd, 1H), 4.79 (t, 2H), 3.84 (t, 2H), 3.69 (d, 1H), 3.47 (dt, 1H), 3.23 (d, 1H), 3.07 (q, 3H), 2.88 (t, 2H), 2.20 (dt, 1H), 2.14-2.05 (m, 2H), 1.97 (s, 1H), 1.85 (q, 1H).

## Synthesis Example 3. Synthesis of Compound CPD06

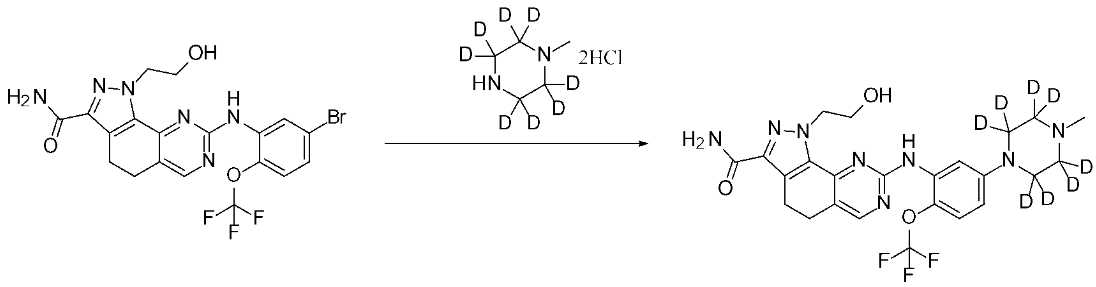


11

CPD 06

A mixture of Compound 11 (2.0 g, 3.9 mmol, 1.0 eq), N-methylpiperazine-2,2,3,3,5,5,6,6-D8 hydrochloride (1.13g, 6.25mmol, 1.6 eq), $Pd_2(dba)_3$ (285 mg, 0.312 mmol, 0.08 eq) and Davephos (123mg, 0.312mmol, 0.08 eq) were added with THF (100 mL) and LiHMDS (40 mL, 40 mmol). The mixture was reacted at 100°C for 2 hours, water was added to quench the reaction, the reaction solution was extracted with EtOAc (50 mL×3), the organic phases were combined, the solvent was removed by rotary evaporation, and the residue was purified by silica gel column chromatography to obtain the target product CPD06 (1g, 47.4%) as a white solid. LCMS: 541.3 $[M+H]^+$, $^1H$ NMR (400 MHz, DMSO-$d_6$) δ ppm 8.92 (s, 1H), 8.34 (s, 1H), 7.45 (bs, 1H), 7.27-7.21 (m, 3H), 6.80 (dd, 1H), 4.62 (q, 3H), 3.62 (q, 2H), 2.97 (t, 2H), 2.79 (t, 2H), 2.49-2.32 (m, 3H).

**Test Example 1. Pharmacokinetic Study in Mice**

The objective of this study is to investigate the pharmacokinetic properties of the disclosed compounds after tail vein intravenous injection (IV) and oral administration (PO) in female CD1 mice. Plasma was sampleed at 0, 0.083, 0.25, 0.5, 1, 2, 4, 7 and 24 hours post-dosing, and the drug concentrations in plasma at different time points were detected by LC-MS/MS, and the relevant pharmacokinetic parameters were calculated using WinNonlin software.

**1.1 Test Materials**

1.1.1 The reagents and instruments used in this example are as follows:

**Table 2. Reagent Information**

| Reagents | Catalog No. | Batch No. | Brand |
|---|---|---|---|
| DMSO | A00878 | KDGLA04 | Innochem |
| SBE-β-CD | C830138 | C14707121 | Macklin |
| MC | M0262 | SLCL7954 | Sigma-Aldrich |
| Tween-20 | P1379 | WXBD5553V | Sigma-Aldrich |
| NMS-P937 | S81189 | A03IS21133 | Shanghai yuanye Bio-Technology Co., Ltd. |

**Table 3. Instrument Information**

| Instrument | Brand | Model |
|---|---|---|
| Liquid Chromatograph | Waters | ACQUITY SHIMADZU LC-30AD |
| Mass Spectrometer | Waters | Xevo TQ-S Micro Sciex Triple Quad 5500 |
| Micro Analytical Balance | Sartorius Scientific Instruments (Beijing) Co., Ltd. | SQP |
| Vortex Mixer | IKA | IKA MS3 basic |
| High-Speed Refrigerated Centrifuge | Eppendorf China Co., Ltd. | 5810R |

1.1.2 Experimental Animals

Experimental female CD1 mice were purchased from SPF Laboratory Animal Technology Co., Ltd., aged 6-8

weeks with body weights of 20-30 g. The female CD1 mice were housed in a controlled environment (set temperature at 20-25°C and relative humidity at 40-70%). A 12-hour light/12-hour dark cycle was maintained unless interrupted by study-related events.

### 1.2 Experimental Procedures

1.2.1 Preparation of Experimental Compound Solutions

The positive control compound used in this example is NMS-P937, which has the structure shown in the following formula:

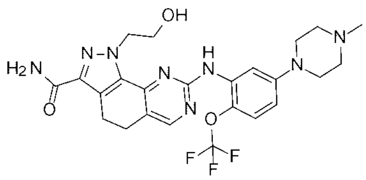

NMS-P937

Compound solutions are prepared in an ultra-clean workbench and prepared immediately before use, and mixed thoroughly prior to use to ensure formulation homogeneity. The specific compound preparation method is shown in the table below:
Preparation of 5 mg/mL stock solutions of compounds CPD02 and CPD03

| Test Compound | Concentration (mg/mL) | Mass (mg) | DMSO (mL) |
|---|---|---|---|
| CPD02 | 5 | 1.18 | 0.234 |
| CPD03 | 5 | 1.37 | 0.271 |
| NMS-P937 | 5 | 1.33 | 0.263 |

Preparation of 20 mg/mL stock solutions of compounds CPD04, CPD06 and NMS-P937

| Test Compound | Concentration (mg/mL) | Mass(mg) | DMSO (mL) |
|---|---|---|---|
| CPD04 | 20 | 1.79 | 0.090 |
| CPD06 | 20 | 1.87 | 0.091 |
| NMS-P937 | 20 | 2.06 | 1.03 |

1.2.2 Administration

Administration method: IV

Vehicle composition: 10% DMSO+90% (20%SBE-β-CD)

**Table 4. Doses Used in IV Administration**

| Test Compound | Dose (mg/kg) | Volume (mL/kg) | Concentrati on (mg/mL) | Stock Solution (mL) | DMSO (mL) | 20% SBE-β-CD (mL) |
|---|---|---|---|---|---|---|
| CPD02 | 2 | 5 | 0.4 | 0.080 | 0.020 | 0.900 |
| CPD03 | 2 | 5 | 0.4 | 0.096 | 0.024 | 1.080 |
| NMS-P937 | 2 | 5 | 0.4 | 0.096 | 0.024 | 1.080 |
| CPD04 | 10 | 5 | 2 | 0.935 | 0.094 | 0.842 |
| CPD06 | 10 | 5 | 2 | 0.895 | 0.090 | 0.806 |

(continued)

| Test Compound | Dose (mg/kg) | Volume (mL/kg) | Concentrati on (mg/mL) | Stock Solution (mL) | DMSO (mL) | 20% SBE-β-CD (mL) |
|---|---|---|---|---|---|---|
| NMS-P937 | 10 | 5 | 2 | 1.030 | 0.103 | 0.773 |

Administration method: PO

Vehicle composition: 10% DMSO + 90% (20%SBE-β-CD)

**Table 5. Doses Used in PO Administration**

| Test Compound | Dose (mg/kg) | Volume (mL/kg) | Concentration (mg/mL) | Stock Solution (mL) | 20% SBE-β-CD (mL) |
|---|---|---|---|---|---|
| CPD02 | 5 | 10 | 0.5 | 0.100 | 0.900 |
| CPD03 | 5 | 10 | 0.5 | 0.120 | 1.080 |
| NMS-P937 | 5 | 10 | 0.5 | 0.120 | 1.080 |

Vehicle composition: 0.5% MC + 1% Tween-20

**Table 6. Doses Used in PO Administration**

| Test Compound | Dose (mg/kg) | Volume (mL/kg) | concentration (mg/mL) | Stock solution (mL) | 0.5% MC +1% Tween-20 (mL) |
|---|---|---|---|---|---|
| CPD04 | 10 | 10 | 1 | 1.510 | 1.510 |
| CPD06 | 10 | 10 | 1 | 1.430 | 1.430 |
| NMS-P937 | 10 | 10 | 1 | 1.440 | 1.440 |

1.2.3 Drug administration and blood sample collection protocol

Prior to the experiment, the animals were randomly grouped. The mice were fasted for 12 hours before drug administration, and free feeding was resumed 4 hours after administration. The animal grouping and blood sampling schedule are shown in the table below.

**Table 7. Animal Grouping and Blood Sampling Schedule**

| Compound | Administration Method | Dose (mg/kg) | Concentration (mg/mL) | Volume (mL/kg) | Number of animals (n) | Blood sampling time points |
|---|---|---|---|---|---|---|
| CPD 02, CPD 03 and NMS-P937 | IV | 2 | 0.4 | 5 | N=3 | 0 min, 5 min, 15 min, 30 min, 1 h, 2 h, 4 h, 7 h, 24 h |
| | PO | 5 | 0.5 | 10 | N=3 | 0 min, 5 min, 15 min, 30 min, 1 h, 2 h, 4 h, 7 h, 24 h |
| CPD 04, CPD 06 and NMS-P937 | IV | 10 | 1 | 10 | N=3 | 0 min, 5 min, 15 min, 30 min, 1 h, 2 h, 4 h, 7 h, 24 h |
| | PO | 10 | 1 | 10 | N=3 | 0 min, 5 min, 15 min, 30 min, 1 h, 2 h, 4 h, 7 h, 24 h |

All blood samples were transferred into plastic microcentrifuge tubes containing anticoagulant (EDTA-$K_2$), and then centrifuged at 4000 g for 5 minutes at 4°C. The supernatant was transferred into a microcentrifuge tube without anticoagulant, and the plasma was stored in a -75±15°C refrigerator until detection.

### 1.3 Data and Results

The blood samples were purified by HPLC and analyzed by mass spectrometry to obtain the plasma concentration-time curve of the disclosed compound.

Among them, for compounds CPD02 and CPD03, the Waters ACQUITY HPLC apparatus was used, and the HPLC purification conditions are shown in the table below:

**Table 8. HPLC Purification Conditions**

| | | | |
|---|---|---|---|
| Chromatographic column: | XSelect Hss T3 2.5μm (2.1 × 50 mm) Column XP | | |
| Column temperature: | 40°C | | |
| Run time: | 2 min | | |
| Mobile phase A: | Water (containing 0.1% formic acid) | | |
| Mobile phase B: | Acetonitrile (containing 0.1% formic acid) | | |
| Injection volume: | 10μL | | |
| **Time (min)** | **Flow rate (mL/min)** | **Water (containing 0.1% FA)** | **Acetonitrile (containing 0.1% FA)** |
| Initial | 0.5 | 95.0 | 5.00 |
| 0.30 | 0.5 | 95.0 | 5.00 |
| 0.80 | 0.5 | 5.00 | 95.0 |
| 1.20 | 0.5 | 5.00 | 95.0 |
| 1.50 | 0.5 | 95.0 | 5.00 |
| 2.00 | 0.5 | 95.0 | 5.00 |

Among them, for compounds CPD 04 and CPD06, AB SHIMADZU LC-30AD HPLC apparatus was used, and the HPLC purification conditions are shown in the table below:

**Table 9. HPLC Purification Conditions**

| | | | |
|---|---|---|---|
| Chromatographic column: | XSelect Hss T3 2.5μm (2.1 × 50 mm) Column XP | | |
| Column temperature: | 40°C | | |
| Run time: | 2 min | | |
| Mobile phase A: | Water (containing 0.1% formic acid) | | |
| Mobile phase B: | Acetonitrile (containing 0.1% formic acid) | | |
| Injection volume: | 10μL | | |
| **Time (min)** | **Flow rate (mL/min)** | **Water (containing 0.1% FA)** | **Acetonitrile (containing 0.1% FA)** |
| initial | 0.8 | 95.0 | 5.00 |
| 0.30 | 0.8 | 95.0 | 5.00 |
| 1.00 | 0.8 | 2.00 | 98.0 |
| 1.48 | 0.8 | 2.00 | 98.0 |
| 1.51 | 0.8 | 95.0 | 5.00 |
| 2.00 | 0.8 | 95.0 | 5.00 |

The pharmacokinetic parameters were estimated by non-compartmental model method (calculated by Phoenix WinNonlin software). IV pharmacokinetic parameters included CL, $T_{1/2}$, $C_0$, AUC, MRT, Vss; PO pharmacokinetic parameters included $T_{1/2}$, $T_{max}$, $C_{max}$, AUC, MRT, etc.

The formula for calculating bioavailability F is as follows:

$$F=\left[\frac{AUC_{PO}\times Dose_{IV}}{AUC_{IV}\times Dose_{PO}}\right]\times 100\%$$

The pharmacokinetic parameters of the disclosed compounds obtained in this example are summarized as follows:

**Table 10. Pharmacokinetic Parameters of SD Mice After Intravenous Administration**

| Compound | Dose (mg/kg) | $t_{1/2}$ (hr) | $AUC_{last}$ (hr*ng/mL) | Vss (L/kg) | CL (mL/min/kg) |
|---|---|---|---|---|---|
| CPD02 | 2 | 1.36 | 404 | 3.05 | 81.7 |
| CPD03 | 2 | 3.76 | 433 | 4.29 | 79.1 |
| NMS-P937 | 2 | 1.52 | 353 | 5.71 | 95.7 |
| CPD 04 | 10 | 1.06 | 3341 | 2.31 | 50.1 |
| CPD 06 | 10 | 1.05 | 3051 | 3.02 | 55.1 |
| NMS-P937 | 10 | 3.52 | 4539 | 3.22 | 36.9 |

**Table 11. Pharmacokinetic Parameters of SD Mice After Oral Administration**

| Compound | Dose (mg/kg) | $t_{1/2}$ (hr) | $t_{max}$ (hr) | $C_{max}$ (ng/mL) | $AUC_{last}$ (hr*ng/mL) | F (%) |
|---|---|---|---|---|---|---|
| CPD02 | 5 | 2.71 | 0.500 | 169 | 359 | 38.0 |
| CPD03 | 5 | 3.03 | 0.417 | 62.7 | 144 | 16.2 |
| NMS-P937 | 5 | 2.62 | 0.833 | 55 | 167 | 20.5 |
| CPD 04 | 10 | 1.44 | 0.667 | 613 | 1397 | 42.8 |
| CPD 06 | 10 | 1.16 | 0.333 | 510 | 1288 | 43.1 |
| NMS-P937 | 10 | 1.14 | 0.333 | 599 | 1212 | 27.1 |

The pharmacokinetic data show that the disclosed compounds have a suitable half-life in mice, the oral bioavailability of the stereospecific design R-configuration molecule CPD 02 is significantly higher than that of the S-configuration CPD 03, and higher than that of NMS-P937, and the bioavailability of the deuterated compound is significantly higher than that of the non-deuterated molecule (CPD 04 vs. CPD 02, CPD 06 vs. NMS-P937).

**Test Example 2. Proliferation Inhibitory Activity on Cell Line PSN1**

**2.1 Test Materials**

The reagents and instrument information used in this Example are as follows:

**Table 12 Cell Line Information**

| Cell lines | Manufacturer | Culture Medium |
|---|---|---|
| PSN1 | Nanjing Cobioer | RPMI 1640 + 10% FBS |

**Table 13. Main Reagents and Consumables Information**

| Name | Supplier | Catalog No. |
|---|---|---|
| CellTiter-Glo® | Promega | G7572 |
| RPMI-1640 | VivaCell | C3010-0500 |
| DMEM | VivaCell | C3113-0500 |
| IMDM | ATCC | ATCC®30-2005 |

(continued)

| Name | Supplier | Catalog No. |
|---|---|---|
| Fetal bovine serum | ExCellBio | FND500 |
| Insulin | BasalMedia | S454T0 |
| Trypsin (2.5%) | Gibco | 15090-046 |
| Horse serum | LIFE | 16050122 |
| 96-well plate | Agilent Technologies | 204626-100 |

**Table 14. Instrument and Software Information**

| Catalog | Supplier | Catalog No. |
|---|---|---|
| Incubator | RADOBIO | Herocell 180 |
| Biological Safety Cabinets | AIRTECH | BSC-1304 II A2 |
| Automated Cell Counter | Thermo | Countess III |
| Multimode Reader | PE | Ensight |
| GraphPad Prism 8.0 Software | / | / |

**Table 15. Compound Information and Stock Solution Preparation**

| Compound | MW | Mass (mg) | Stock Concentration (mM) | DMSO Volume (μL) |
|---|---|---|---|---|
| CPD02 | 558.57 | 1.10 | 20 | 98.5 |
| CPD03 | 558.57 | 1.08 | 20 | 96.7 |
| CPD04 | 562.59 | 2.01 | 20 | 178.6 |
| CPD06 | 540.58 | 2.1 | 20 | 194.2 |
| NMS-P937 | 532.53 | 2 | 20 | 187.8 |

**2.2 Test Methods**

Day 0: Plating

a) Adherent cells need to be digested and centrifuged, while suspension cells can be directly centrifuged;
b) Resuspend with complete culture medium and count using an automatic cell counter;
c) Plate 6,000 cells per well for PSN1, and dilute the cell suspension to the required density;
d) Add 100 μL of cells per well, and culture overnight at 37°C in a 5% $CO_2$ incubator to stabilize and balance the cells.

Day 1: Compound Preparation and Addition

a) Prepare the compound with DMSO as a solution of 200-fold final concentration;
b) Dilute the 200-fold compound to a 3-fold final concentration with complete culture medium;
c) Add 50 μL of the compound per well, use wells with the same volume of culture medium as controls, and continue culturing in a 37°C, 5% $CO_2$ incubator for 72 hours.

Day 4: Detection

a) Equilibrate the cell plate and CTG reagent to room temperature;

b) Add 30 μL of CellTiter-Glo® reagent per well;
c) Incubate at room temperature for 15 minutes;
d) Detect with a microplate reader.

Data Analysis

1) Calculate $IC_{50}$ using GraphPad Prism 8;
2) %Inh = (1-(Raw data - $Mean_{min}$) / ($Mean_{max}$ - $Mean_{min}$))×100;
3) $Mean_{max}$ is the positive control well with only cells and the same volume of culture medium;
4) $Mean_{min}$ is the negative control well with only culture medium;
5) Raw data refers to the signal values of wells with added compounds. For two replicate wells, they are denoted as Raw1 and Raw2.

**2.3 Test Results**

The half maximal inhibitory concentration ($IC_{50}$) of the compounds of the present disclosure on PSN1 cell proliferation is shown in Table 16.

**Table 16. $IC_{50}$ of the Compounds of the Present Disclosure on PSN1 Cell Proliferation**

| Compound No. | Max Inhibition Percentage | Max Inhibition Percentage | $IC_{50}$ (nM) |
|---|---|---|---|
| CPD02 | 80.8 | 6.7 | 67.6 |
| CPD03 | 73.6 | -3.1 | 255.9 |
| CPD04 | 87.6 | 1.3 | 105.0 |
| CPD06 | 93.7 | 13.7 | 57.0 |
| NMS-P937 | 85.9 | 2.3 | 53.7 |

It can be concluded from the cell proliferation inhibition test that the compounds of the present disclosure exhibit significant growth inhibitory effects on the pancreatic cancer cell line (PSN1). The stereospecific design R-configured molecule CPD02 shows higher sensitivity than the S-configured CPD03. Deuterated compounds and non-deuterated molecules show no significant difference in biological activity (PSN1) (e.g., CPD04 vs. CPD02, CPD06 vs. NMS-P937).

**Test Example 3. Metabolic Stability Evaluation in Liver Microsomes**

**3.1 Test materials**

The materials used in this example are shown in Tables 17-19 below:

**Table 17. Compound Information**

| Compound Name | Mass | Molecular Weight | Concentrat ion | Solvent (μL) -DMSO | Preparation Date |
|---|---|---|---|---|---|
| CPD 04 | 1.80 mg | 562.59 | 10 mM | 320 | 20230510 |
| CPD 06 | 3.13 mg | 540.58 | 10 mM | 579 | 20230510 |
| NMS-P937 | 2.65 mg | 523.53 | 10 mM | 498 | 20230510 |
| CPD 02 | 1.73 mg | 558.57 | 10 mM | 310 | 20230510 |

**Table 18. Microsome Information**

| Name | Brand or supplier | Catalog No. | Batch No. |
|---|---|---|---|
| Human Liver Microsomes, Male and Female | Gentest | 452117 | 38297 |
| Male Mouse Liver Microsomes, Male CD-1 | Gentest | 452701 | 1313002 |

**Table 19. Reagent Information**

| Name | Brand or supplier | Catalog No. | Batch No. |
|---|---|---|---|
| NADPH | Solarbio | N8100 | 812P021 |
| Ketoprofen | MCE | HY-B0227 | 105978 |
| Labetalol hydrochloride | MCE | HY-B1108 | 108780 |
| Alprazolam | Sigma | A-903-1ML | FE12172003 |
| MeOH | Fisher | A452-4 | 213476 |
| ACN | Honeywell | LC015-1-1 | EB072-CN |
| DMSO | Solarbio | D8372 | 1121C0310 |
| $MgCl_2$ | Solarbio | M8161 | 111D031 |
| UDPGA | yuanye | S18073 | O11GS162290 |
| NADPH* | solarbio | N8100 | 812P021 |
| Alamethicin | MCE | HY-N6708 | 133878 |

**3.2 Test Methods**

3.2.1 Reagent Preparation

| Name | Stock Concentration | Volume (μL) | Final Concentration |
|---|---|---|---|
| Phosphate Buffer Saline | 100 mM | 266 | 100 mM |
| $MgCl_2$ | 50 mM | 40 | 5 mM |
| NADPH | 10 mM | 40 | 1 mM |
| UDPGA | 20 mM | 40 | 2 mM |
| Liver Microsomes | 20 mg/mL | 10 | 0.5 mg/mL |
| Alamethicin | 5 mg/mL | 2 | 0.025 mg/mL |
| Test compound | 400 μM | 2 | 2 μM |
| Total | | 400 | |

3.2.2 Experimental Procedures

1) Prepare a microsome solution at 0.6289 mg/mL using PBS.
2) Add 318 μL of the 0.6289 mg/mL microsome solution to each well of a 96-well incubation plate.
3) Add 40μL of 10mM NADPH solution and 40μL of 20mM UDPGA solution to the incubation plate. For negative samples, add 80μL of PBS (100mM, pH = 7.4) solution. Vortex at 800 rpm for 10 seconds, then pre-incubate in a 37°C water bath for 10 minutes.
4) After pre-incubation, add 2 μL of control drug or test compound to each well to initiate the reaction. The reaction is carried out in a 37°C water bath at 60 rpm.
5) At 0.5, 15, 30, 45, and 60 minutes, take 50 μL of the reaction system from each well and add it to 200 μL of cold methanol solution containing the internal standard to terminate the reaction.
6) Mix thoroughly and centrifuge at 4000 rpm at 4°C for 30 minutes.
7) Take 100μL of supernatant and mix with 100μL ultrapure water for liquid-mass analysis.

*Internal standards: 50nM alprazolam, 50nM labetalol and 100nM ketoprofen

3.2.3 Data Processing

The internal standard method is adopted, and the ratio of the sample peak area to the internal standard peak area is used to calculate the residual percentage (taking the ratio of the sample peak area to the internal standard peak area at 0.5 minutes as 100%).

The natural logarithm of the residual percentage is plotted against the incubation time, and the slope of the straight line is the elimination rate constant k.

$$t_{1/2}(\text{min}) = -\frac{0.693}{k}$$

$$Cl_{int}(\mu L/\text{min/mg protein}) = \frac{\text{Incubation Volume }(\mu L)}{\text{Incubation Protein (mg)}} \times \frac{0.693}{t_{1/2}}$$

**3.3 Test Results**

The evaluation results of the metabolic stability of the disclosed compound in liver microsomes are shown in Table 20.

**Table 20. Summary of Liver Microsomal Metabolic Stability Data**

| **Compound** | **Species** | **Residual Amount (%)** | | | | | | $CL_{int}$ |
|---|---|---|---|---|---|---|---|---|
| | | **0.5 min** | **15 min** | **30 min** | **45 min** | **60 min** | $T_{1/2}$ **(min)** | **(μL/min/mg protein)** |
| Diclofenac | Human | 100.00 | 10.78 | 5.62 | 3.65 | 2.93 | 7.13 | 194.50 |
| | | 100.00 | 105.89 | 102.20 | 104.38 | 91.57 | | |
| Diclofenac | Mouse | 100.00 | 61.24 | 56.66 | 51.87 | 45.26 | 20.49 | 67.63 |
| | | 100.00 | 114.45 | 108.86 | 109.75 | 114.70 | | |
| CPD04 | Human | 100.00 | 52.72 | 28.62 | 21.10 | 13.41 | 20.95 | 66.17 |
| | | 100.00 | 86.71 | 108.16 | 107.82 | 102.03 | | |
| CPD04 | Mouse | 100.00 | 61.30 | 39.18 | 34.10 | 26.56 | 31.93 | 43.41 |
| | | 100.00 | 103.15 | 96.04 | 101.86 | 109.33 | | |
| CPD06 | Human | 100.00 | 86.05 | 73.64 | 68.37 | 63.15 | 89.92 | 15.41 |
| | | 100.00 | 93.25 | 90.15 | 89.75 | 91.37 | | |
| CPD06 | Mouse | 100.00 | 81.80 | 68.21 | 64.96 | 57.01 | 76.33 | 18.16 |
| | | 100.00 | 87.75 | 92.85 | 96.22 | 89.24 | | |
| NMS-P937 | Human | 100.00 | 80.23 | 73.16 | 69.74 | 58.94 | 86.35 | 16.05 |
| | | 100.00 | 102.54 | 96.00 | 101.77 | 90.35 | | |
| NMS-P937 | Mouse | 100.00 | 82.56 | 69.31 | 61.60 | 53.17 | 66.38 | 20.88 |
| | | 100.00 | 93.89 | 92.17 | 88.22 | 85.68 | | |
| CPD02 | Human | 100.00 | 54.16 | 32.53 | 22.97 | 13.71 | 21.38 | 64.82 |
| | | 100.00 | 103.64 | 103.22 | 102.41 | 97.08 | | |
| CPD02 | Mouse | 100.00 | 56.76 | 39.33 | 30.29 | 26.56 | 31.53 | 43.95 |
| | | 100.00 | 103.87 | 96.01 | 95.65 | 88.91 | | |
| Conclusion: The disclosed compound shows moderate *in vitro* clearance. | | | | | | | | |

**Test Example 4. Evaluation of Test Substance Permeability and Transporter Substrate Test**

**4.1 Test Materials**

4.1.1 Compound Information

| Compound Name | Mass | Batch No. | Molecular Weight | Concen tration | Solvent (μL) DMSO | Preparation Date |
|---|---|---|---|---|---|---|
| CPD 04 | 1.80 mg | - | 562.59 | 10 mM | 320 | 2023/05/10 |
| CPD 06 | 3.13 mg | - | 540.58 | 10 mM | 579 | 2023/05/10 |
| NMS-P937 | 2.65 mg | - | 532.53 | 10 mM | 498 | 2023/05/10 |
| CPD02 | 1.73 mg | - | 558.57 | 10 mM | 310 | 2023/05/10 |

4.1.2 Positive Control Drug Information

| Compound | Catalog No. | Batch No. | Source | Molecular Weight | Working Fluid Concentration |
|---|---|---|---|---|---|
| **Propranolol** | HY-B0573 | 26496 | MCE | 295.80 | 10 mM |
| **Digoxin** | HY-B1049 | 121301 | MCE | 780.94 | 10 mM |
| **Prazosin** | HY-B0193A | 15874 | MCE | 419.86 | 10 mM |

**4.2 Test Methods**

4.2.1 Caco-2 Cell Culture

a) Add 50 μL and 25 mL of cell culture medium to the upper chamber and lower chamber of each well of the Transwell plate, respectively;
b) Pre-incubate the Transwell plate at 37°C, 5% $CO_2$ for 1 hour before seeding the cells;
c) Seed 50 μL of cell suspension at a density of $2 \times 10^5$ cells/mL in the upper chamber, and culture in a cell culture incubator at 37°C, 5% $CO_2$, and 95% relative humidity for 14-21 days, replacing the medium every two days;
d) Replace the cell culture medium every other day within 7 days, and daily after 7 days.
e) Measure the electrical resistance (TEER) of the monolayer using an EVOM3.

4.2.2 ABBA Testing Process

a) Wash the transwell plate twice with pre-warmed HBSS (10 mM HEPES, pH 7.4) and then incubate at 37°C for 30 minutes;
b) Prepare a 1 mM DMSO stock solution of the test compound and dilute it 200-fold to 5 μM with buffer (pH 7.2-7.4);
c) A to B direction: add 75 μL of 5 μM working solution to the upper chamber and 235μL of HBSS (10mM HEPES, pH 7.4) to the lower chamber;
d) B to A direction: add 235μL 5μM working solution to the lower chamber and 75μL HBSS (10mM HEPES, pH 7.4) to the upper chamber;
e) Transfer 50 μL of the 5 μM working solution to the sample plate containing 200 μL of pre-chilled methanol internal standard as the zero point;
f) Incubate at 37°C, 5% $CO_2$, 95% relative humidity for 2 hours;
g) After 2 hours of incubation, transfer 50 μL from each well to the sample plate containing 200 μL of methanol internal

standard;

h) Centrifuge the sample plate at 4000 rpm for 30 minutes;

i) Transfer 100 μL of the supernatant to an assay plate containing 100 μL of water and centrifuge for 10 min before LC-MS/MS analysis.

4.2.3 Data Analysis

Apparent permeability coefficient (Papp, cm/s) and efflux ratio were calculated using Microsoft Excel.

$$P_{app} = \frac{V_A \times C_{acceptor} \times 100}{area \times time \times C_{initial,donor}}$$

$$\text{Efflux ratio} = \frac{P_{app(B-A)}}{P_{app(A-B)}}$$

$$\text{Recovery\%} = \frac{V_{acceptor} \times C_{acceptor} + V_{donor} \times C_{donor}}{V_{donor} \times C_{initial,donor}} \times 100$$

$V_{acceptor}$ = 0.235 mL (for A→B) and 0.075 mL (for B→A)

$V_{donor}$ = 0.075 mL (for A→B) and 0.235 mL (for B→A)

Area = membrane surface area, $cm^2$ (0.143 for Corning 3391)

Time = total transport time, s

4.3 Classification Criteria

| Parameter | Classification | Criteria |
|---|---|---|
| Permeability | Low permeability | Papp (A -B) < 2E-6 cm/s |
| | Moderate Permeability | 2E-6 cm/s <Papp (A -B) < 20E-6 cm/s |
| | High Permeability | Papp (A -B)> 20E-6 cm/s |
| Efflux Transporter Substrate | Probable | ERa ≧ 2 |
| | Unlikely or Not | ERa<2 |

**4.4 Test Results**

The permeability results of the disclosed compounds in Caco-2 cells are shown in Table 21.

**Table 21. Permeability Results in Caco-2 cells**

| | Average $P_{app}$ (1E-6 cm/s) | | | | Classification |
|---|---|---|---|---|---|
| **Compound** | **$Papp_A$-B** | **$Papp_B$-A** | **Efflux Rate (ERa)** | **Permeability** | **Efflux Transporter Substrate** |
| **Propranolol** | 22.32 | 14.73 | 0.66 | 61.61 | Unlikely or Not |
| **Digoxin** | 0.94 | 26.07 | 27.84 | 78.46 | Probable |
| **Prazosin** | 8.97 | 29.57 | 3.30 | 71.80 | Probable |
| CPD 04 | 4.20 | 25.15 | 5.98 | 53.35 | Probable |
| CPD 06 | 5.18 | 34.74 | 6.71 | 79.65 | Probable |
| NMS-P937 | 6.09 | 34.16 | 5.61 | 71.57 | Probable |

(continued)

| | Average $P_{app}$ (1E-6 cm/s) | | | | Classification |
|---|---|---|---|---|---|
| Compound | $Papp_A$-B | $Papp_B$-A | Efflux Rate (ERa) | Permeability | Efflux Transporter Substrate |
| CPD 02 | 7.83 | 23.78 | 3.04 | 67.07 | Probable |

**Test Example 5. Evaluation of Solubility Test for Test Substances**

**5.1 Test Materials**

| Name | Brand or Supplier | Catalog No. | Batch No. |
|---|---|---|---|
| PBS | ICE | - | 20230509 |
| Progesterone | MCE | 57-83-0 | 16163 |
| FaSSIF/FeSSIF/FaSSGF Powder | Biorelevant | FFF02 | FFF-0421-E |
| 1.5mL MTP System-(Topas) | JG Finneran | 9915LC-812FT | 436021308MTP |
| DMSO | Solarbio | D8372 | 1121C0310 |

**5.2 Test Methods**

5.2.1 Preparation of Solution (Buffer)

Weigh 7.098 g $Na_2HPO_4$ (Solution A) and dissolve in 500 mL ultrapure water via sonication; weigh 3.40 g $KH_2PO_4$ (Solution B) and dissolve in 250 mL ultrapure water via sonication. Slowly add Solution B dropwise to Solution A and adjust the pH to 7.40 ± 0.05.

5.2.2 Test Procedures

a) Weigh approximately 1.0 mg of compound into three separate 1.5 mL glass vials (one vial for preparing the standard solution and the other two for testing solubility);
b) Add an appropriate volume (approximately 1000 μL) of assay buffer to each solubility sample vail to a final concentration of 1 mg/mL;
c) Place a PTFE-encapsulated stainless steel rod stirrer into each vial and seal the vials using PTDE/SIL silicone stoppers;
d) Transfer the solubility sample plate to a Thermomixer Comfort plate shaker and shake at 1100 rpm, 25°C for 24 hours;
e) After 24 hours, remove the stirrers using a magnet;
f) Pipette 200 μL of sample into the filter plate, perform vacuum filtration, and collect the filtrate;
g) Add 10 μL of the filtered sample and 10 μL of DMSO to 980 μL of methanol, then further dilute 10-fold with methanol:water (1:1) as the filtered sample for LC-MS/MS analysis;
h) For the standard sample, add DMSO to a final concentration of 1.0 mg/mL. Shake at 1100 rpm, 25°C for 5 minutes;
i) Add 10 μL of the 1 mg/mL DMSO sample and 10 μL of buffer to 980 μL of methanol, then dilute 10-fold with methanol:water (1:1) as the standard sample for LC-MS/MS analysis.

5.2.3 Data Processing

Solubility was calculated using Excel.

$$\text{Solubility}\ (\mu\text{M}) = \frac{Area_{\text{filtered}} \times DF_{\text{filtered}}}{Area_{\text{std}} \times DF_{\text{std}}} \times [\text{std}] \times DF_{\text{std}}$$

### 5.3 Test Results

The solubility results of the disclosed compounds are shown in Table 22.

**Table 22 Summary of Thermodynamic Solubility Data**

| Compound | Solvents | Solubility (mg/mL) |
|---|---|---|
| CPD04 | pH 7.4 | 0.0030 |
| CPD06 | pH 7.4 | 0.0226 |
| NMS-P937 | pH 7.4 | 0.0248 |
| CPD 02 | pH 7.4 | 0.0021 |

### 5.4 Test Conclusion

The compounds of the present disclosure exhibit solubility comparable to that of NMS-P937.

### Test Example 6. Pharmacodynamic Study of Compound on Colorectal Cancer in Mice

### 6.1 Test Materials

Balb/c nude mice: SPF grade, female, 6-8 weeks old, weighing 16-20 g, purchased from SPF (Beijing) Biotechnology Co., Ltd.
HCT116 cells: purchased from ATCC;

The reagents and instruments used in the *in vivo* pharmacodynamic experiments are shown in Tables 23 and 24.

**Table 23. Reagents Used in *In Vivo* Pharmacodynamic Experiments**

| Name | Specification | Manufacturer | Batch No. |
|---|---|---|---|
| PBS (Phosphate Buffered Saline) | 500 mL/bottle | Dalian Meilun Biotechnology Co., Ltd. | MA0015-Sep-27G |
| RPMI1640 Medium | 500 mL/bottle | Dalian Meilun Biotechnology Co., Ltd. | MA0215-Dec-17G |
| Fetal Bovine Serum (FBS) | 500mL/bottle | ExcellBio | 12A222 |
| Trypsin-EDTA Digestion Solution (0.25%) | 100 mL/bottle | Solarbio | 20220328 |
| Penicillin-Streptomycin | 100 mL/bottle | Solarbio | 20211027 |
| Sterile Water for Injection | 500 mL/bottle | Dalian Meilun Biotechnology Co., Ltd. | PWL093-Mar-25H |

**Table 24. Instruments and Equipment Used in *In Vivo* Pharmacodynamic Experiments**

| Name | Model | Manufacturer | Application |
|---|---|---|---|
| $CO_2$ Incubator | 3111 | Thermo | Cell culture |
| Microscope | IX53 | Olympus | Cell culture |
| Ultra-Clean Workbench | YT-CJ-1N | Beijing Yatai Kelong Instrument Technology Co., Ltd. | Cell culture |
| Ultra-Clean Workbench | SW-CJ-1FD | Suzhou Purification Equipment Co., Ltd. | Dispensing |
| Ultra-Clean Workbench | YT-CJ-2NH | Beijing Yatai Kelong Instrument Technology Co., Ltd. | *In vivo* experimental operation |

(continued)

| Name | Model | Manufacturer | Application |
|---|---|---|---|
| Electronic Balance | EJ-1201C | Beijing Langke Xingye Weighing Equipment Co., Ltd. | *In vivo* experimental operation |

**6.2 Test Methods**

6.2.1 Cell Culture

HCT116 cells were cultured in RPMI-1640 medium containing inactivated 10% fetal bovine serum, 100 U/mL penicillin, 100 μg/mL streptomycin and 2 mM glutamine in an incubator at 37°C with 5% $CO_2$. Once the cells reached confluence, they were subcultured into new flasks. The tumor cells in the logarithmic growth phase were used for inoculation of tumors *in vivo.*

6.2.2 Tumor Cell Inoculation and Grouping

The tumor cells were washed twice with PBS, and the cell concentration was adjusted to $5\times10^7$/ml. The resuspended tumor cells were inoculated subcutaneously in the right flank of the experimental animals at 100 μL/mouse, i.e. $5\times10^6$/mouse. When the tumor grew to an average tumor volume of 100-150 $mM^3$, the animals were grouped for drug administration according to the tumor volume, with a total of 7 groups, 8 animals in each group. The specific administration schedule is shown in Table 25. The first day of group drug administration was recorded as PG-D0.

**Table 25. Administration Schedule**

| Group | Number of Animals | Treatment | Dose (mg/kg) | Route of Administration | Dosing Frequency | Dosing Cycle |
|---|---|---|---|---|---|---|
| 1 | 8 | Vehicle | -- | p.o. | q.d. | 10 consecutive days |
| 2 | 8 | CPD04 | 30 | p.o. | q.d. | 10 consecutive days |
| 3 | 8 | CPD04 | 60 | p.o. | q.d. | 10 consecutive days |
| 4 | 8 | CPD06 | 30 | p.o. | q.d. | 10 consecutive days |
| 5 | 8 | CPD06 | 60 | p.o. | q.d. | 10 consecutive days |
| 6 | 8 | NMS-P937 | 30 | p.o. | q.d. | 10 consecutive days |
| 7 | 8 | NMS-P937 | 60 | p.o. | q.d. | 10 consecutive days |
| Note: The administration volume is 10 μl /g. p.o.: administration by oral gavage. q.d.: administration once daily for 10 consecutive days. | | | | | | |

6.2.3 Dose Adjustment or Suspension

During the administration, the health status of the animals is monitored. If any of the following conditions occur, the administration will be suspended:

1) If the animal's body weight decreases to 85% or less of the weight at the start of drug treatment, administration shall be stopped until the body weight recovers to 90% of that at the start of drug treatment, after which administration may resume;
2) If the animal exhibits progressive slowness or abnormality in behavior after administration (such as hypothermia, dyspnea, diarrhea, hunched posture, convulsions, etc.), administration shall be suspended until the animal's behavior and general condition restore to normal, after which administration may resume.

6.2.4 Humane Endpoints for Experimental Animals

During the experiment, animals shall be euthanized with $CO_2$ if any of the following conditions occur:

1) The animal exhibits abnormal behavior or becomes paralyzed and is unable to eat or drink spontaneously;

2) The animal's body weight decreases by more than 20% of the weight at the start of drug treatment, and the veterinarian believes that it seriously affects the animal's welfare and/or the normal progress of the experiment.

6.2.5 Euthanasia of Experimental Animals

At the end of the experiment or when a humane endpoint is achieved, animals are euthanized using $CO_2$.

6.2.6 Detection Indicators

**General Observations**

From grouping until the end of the experiment, observations were conducted once daily, including but were not limited to the tumor inoculation site, animal mental state, food intake, motor activity, etc.

**Body Weight Measurement**

From grouping until the end of the experiment, mouse body weight was measured every 3 days using an electronic balance.

**Tumor Volume**

From grouping until the end of the experiment, the long and short diameters of the mouse tumors were measured every 3 days using an electronic vernier caliper, and the volume was calculated using the formula: Volume (TV) = 0.5 × long diameter × (short diameter)$^2$.

**Tumor Photography**

At the end of the experiment, the mice were euthanized, the tumors were excised, and the excised tumors from the control group and the test group were neatly arranged for photography.

6.2.7 Drug Evaluation Indicators

**Tumor Growth Inhibition Rate $TGI_{TV}$ (%)**

$$\text{Tumor growth inhibition rate (\%)} = (1\text{-}T/C) \times 100\%$$

$$T/C = \text{Mean RTV of the treatment group / Mean RTV of the control group}$$

RTV is the relative tumor volume, that is, the ratio of the tumor volume after administration to that before administration.

The tumor growth inhibition rate ≥30% and statistical analysis $p<0.05$ was considered effective.

**Tumor Weight Inhibition Rate TGITw (%)**

$$\text{Tumor weight inhibition rate (\%)} = (1\text{-}T/C) \times 100\%$$

$$T/C = \text{Mean TW of the treatment group / Mean TW of the control group}$$

TW is tumor weight

The tumor weight inhibition rate ≥30% and statistical analysis $p<0.05$ was considered effective.

6.2.8 Statistical Analysis

Graphpad prism 8 statistical software was used to perform statistical analysis of tumor volume and tumor weight among groups using the One-Way ANOVA test (homogeneity of variance), and $p < 0.05$ was considered statistically significant.

**6.3 Test Results**

6.3.1 Tumor Inhibitory Effect of Test Drugs on HCT116 Subcutaneous Tumor Xenograft Mouse Model

As shown in Figure 2: After oral administration for 10 days, at the end of the experiment (PG-D22), compared with the vehicle group, the 60 mg/kg CPD04 administration group, and the 30 mg/kg and 60 mg/kg CPD06 and NMS-P937 administration groups could significantly inhibit the growth of mouse tumors. Each dosage group of CPD04, CPD06 and NMS-P937 showed a dose-dependent effect of tumor inhibition. The tumor growth inhibition rates ($TGI_{TV}$) of each group are shown in Table 26

**Table 26. Tumor Growth Inhibition Rates ($TGI_{TV}$) of Each Group at Test Endpoint**

| Treatment | **Dose (mg/kg)** | TGI (%) | Significance (p-value) |
|---|---|---|---|
| CPD04 | 30 | 7.6 | p<0.01 |
| CPD04 | 60 | 30.5 | p<0.0001 |
| CPD06 | 30 | 42.4 | p<0.0001 |
| CPD06 | 60 | 75.6 | p<0.0001 |
| NMS-P937 | 30 | 44.1 | p<0.0001 |
| NMS-P937 | 60 | 64.2 | p<0.0001 |

6.3.2 Safety of the Test Drugs in HCT116 Subcutaneous Tumor Xenograft Mouse Model

As shown in Figure 3, during the treatment, three mice in the NMS-P937 60 mg/kg group died from PG-D12 and PG-D16, respectively. Mice in the NMS-P937 group showed greater body weight fluctuations. The weight loss may be due to the poor tolerance of tumor-bearing mice to NMS-P937. After drug withdrawal, the condition of some animals failed to improve in time and led to death. In other groups, the weight loss during the treatment period was less than 10%.

**6.4 Test conclusion**

In summary, in the HCT116 subcutaneous tumor xenograft mouse model, each dose group of CPD04, CPD06 and NMS-P937 inhibited mouse tumor growth in a dose-dependent manner. The CPD06 60mg/kg administration group had a significantly better tumor growth inhibition effect than other treatment groups, with a $TGI_{TV}$ of 75.6%, and showed a significant toxicity advantage over the same dose of NMS-P937 (3 out of 8 mice died in the same dose of NMS-P937 group).

**Test Example 7. Pharmacodynamic Study of Compounds on Pancreatic Cancer in Mice**

**7.1 Test Materials**

Pancreatic cancer cell line BxPC-3: purchased from ATCC, other test materials and instruments are the same as in Example 6.

**7.2 Test Methods**

The administration schedule is shown in Table 27. Other test methods are the same as those in Example 9.

**Table 27. Administration Schedule**

| Group | Number of Animals | Treatment | Dose (mg/kg) | Route of Administration | Dosing Frequency | Dosing Cycle |
|---|---|---|---|---|---|---|
| 1 | 8 | Vehicle | -- | p.o. | q.d. | 10 consecutive days |
| 2 | 8 | CPD04 | 90 | p.o. | q.d. | 10 consecutive days |
| 3 | 8 | CPD06 | 30 | p.o. | q.d. | 10 consecutive days |
| 4 | 8 | CPD06 | 60 | p.o. | q.d. | 10 consecutive days |
| 5 | 8 | NMS-P937 | 60 | p.o. | q.d. | 10 consecutive days |

**7.3 Test Results**

7.3.1 Tumor Inhibitory Effect of Test Drugs on BxPC-3 Subcutaneous Tumor Xenograft Mouse Model

As shown in Figure 4: After oral administration for 10 days, at the end of the experiment, compared with the vehicle group, each administration group was able to significantly (($p<0.0001$)) inhibit the growth of mouse tumors. Different doses of CPD06 showed a dose-dependent effect on pancreatic tumor inhibition. The tumor growth inhibition rates ($TGI_{TV}$) of each group are shown in Table 28.

**Table 28. Tumor Growth Inhibition Rates ($TGI_{TV}$) of Each Group at Test Endpoint**

| Treatment | **Dose (mg/kg)** | TGI (%) | Mouse Mortality Rate (%) |
|---|---|---|---|
| CPD04 | 90 | 53 | 0 |
| CPD06 | 30 | 33 | 0 |
| CPD06 | 60 | 71 | 0 |
| NMS-P937 | 60 | 61 | 25 |

7.3.2 Safety of the Test Drugs in the BxPC-3 Subcutaneous Tumor Xenograft Mouse Model

As shown in Figure 5, during the treatment, 2 mice in the NMS-P937 60 mg/kg group died from PG-D9 and PG-D19, respectively. Mice in the NMS-P937 60 mg/kg group showed greater body weight fluctuations. The weight loss may be due to the poor tolerance of tumor-bearing mice to NMS-P937. After drug withdrawal, the condition of some animals failed to improve in time and led to death. However, the weight of other groups was relatively stable during the treatment, with a decrease of less than 10%, and the weight recovered quickly after drug withdrawal.

**7.4 Test Conclusion**

In summary, in the BxPC-3 subcutaneous tumor xenograft mouse model, each dose group of CPD06 inhibited mouse tumor growth in a dose-dependent manner. The CPD06 60mg/kg administration group had a significantly better tumor growth inhibition effect than other treatment groups, with the $TGI_{TV}$ reached 71%, showing that the efficacy was significantly better than the same dose of NMS-P937, and the toxicity was significantly better than the same dose of NMS-P937 group. No obvious toxic symptoms were observed during the treatment of CPD06, but 2 of the 8 mice in the same dose of NMS-P937 group died.

**Test Example 8. Pharmacodynamic Study Of Compound On Pancreatic Cancer In Mice**

**8.1 Test Materials**

M-NSG mice: SPF grade, female, 6-8 weeks old, weighing 16-20 g, purchased from Shanghai Model Organisms Technology Co., Ltd.
PSN1 cells: purchased from ATCC;

The instruments used in the *in vivo* pharmacodynamic experiment are shown in Table 29.

**Table 29. Reagents and Instruments**

| Reagent/Instrument Name | Supplier | Catalog No. | Storage Condition |
|---|---|---|---|
| Electronic Balance | Shanghai Jingtian Electronic Instrument Co., Ltd. | JT201N | RT |
| Vernier Caliper | Mitutoyo | 500-171 | RT |
| Disposable Syringe | Shanghai Kindly Enterprise Development Group Co., Ltd. | Batch K20190705 | RT |
| PBS | BIOLOGICAL INDUSTRIES | C3580-0500 | 4°C |

**8.2 Test Methods**

8.2.1 Drug Preparation

| Sample Name | Preparation Method | Working Solution Concentration (Mg/Ml) | Storage Conditions |
|---|---|---|---|
| NMS-P937 30mg/kg | Weigh 15.0 mg of NMS-P937 into a 5.0 mL EP tube, add 2.5 mL of solvent, and mix to obtain a solution with a concentration of 6 mg/mL. | 6 | 2-8°C |
| CPD04 90mg/kg | Weigh 22.5 mg of CPD04 into a 5.0 mL EP tube, add 2.5 mL of solvent, and mix to obtain a solution with a concentration of 9 mg/mL. | 9 | 2-8°C |
| CPD06 60mg/kg | Weigh 15.0 mg of CPD06 into a 5.0 mL EP tube, add 2.5 mL of solvent, and mix to obtain a solution with a concentration of 6 mg/mL. | 6 | 2-8°C |

8.2.2 Cell Culture

PSN1 cells were cultured in an incubator at 37°C and 5% $CO_2$ in RPMI-1640 medium containing 10% inactivated fetal bovine serum. Cells were subcultured by passaging into new culture dishes every 2 to 3 days when they reach confluent.

8.2.3 Tumor Cell Inoculation and Grouping

8.2.3.1 PSN1 cells in the logarithmic growth phase were taken and resuspended in PBS. The cell suspension was inoculated subcutaneously into the right flank of M-NSG mice using a 1mL syringe (inoculated cell count: 5E6/mouse + 30%M). The survival status of the mice and the subcutaneous tumor formation were observed every other day.
8.2.3.2 On the day of grouping, mice were randomly divided into 8 groups with 8 mice in each group based on the average tumor volume (~190 $mm^3$) and mouse body weight;
8.2.3.3 The mice were dosed according to the grouping scheme, all by intragastric administration. The administration volume was 10 mL/kg, once a day for 6 times in total. The hair condition and status of the mice were observed, and the body weight and tumor size were recorded twice a week to calculate TGI%.
8.2.3.4 Grouping of Test Animals

When the average tumor volume reached ~ 190 $mm^3$, mice with moderate individual tumor volume were selected for inclusion in the group. The animals were randomly assigned to experimental groups according to tumor volume and mouse weight, with 8 mice in each group. Administration started one day after grouping. The specific administration regimen is shown in the table below:

| Group | n | Treatment | Dose (mg/kg) | Administration Volume (μL/g) | Administration Route | Schedule |
|---|---|---|---|---|---|---|
| 1 | 8 | 0.5% MC + 1% Tween20 | - | -- | *p.o.* | QD*6 times |
| 2 | 8 | CPD04 | 90 | 10 | *p.o.* | QD*6 times |
| 3 | 8 | CPD06 | 60 | 10 | *p.o.* | QD*6 times |
| 4 | 8 | NMS-P937 | 60 | 10 | *p.o.* | QD*6 times |

8.2.4 Calculation Formulae

Tumor volume (TV)

$$TV=1/2\times a\times b2$$

wherein: a represents the long diameter of the tumor; b represents the short diameter of the tumor.

Relative tumor volume (RTV)

$$RTV=V_t / V_{initial} \times 100(\%)$$

wherein, $V_{initial}$ is the tumor volume measured at the time of group and administration (ie, $d_{initial}$), and $V_t$ is the tumor volume at each measurement.

Relative tumor growth rate T/C (%)

$$T/C\ (\%) = (T_{RTV} / C_{RTV}) \times 100\%$$

wherein, $T_{RTV}$ represents the relative tumor volume of the treatment group, and $C_{RTV}$ represents the relative tumor volume of the vehicle group.

Tumor volume inhibition rate (TGI)

$$TGI = [1- (TV_t - TV_{initial}) / (CV_t - CV_{initial})] \times 100\%$$

wherein, $TV_t$ represents the tumor volume of the treatment group at each measurement; $TV_{initial}$ represents the tumor volume of the treatment group at the time of grouping and administration; $CV_t$ represents the tumor volume of the control group at each measurement; $CV_{initial}$ represents the tumor volume of the control group at the time of grouping and administration.

8.2.5 Animal Body Weight Change Rate (BWC)

$$\text{Body Weight Change Rate} = (BW_{final} - BW_{initial}) / BW_{initial} \times 100\%$$

wherein, BWinitial represents the animal body weight at the time of grouping and administration; $BW_{final}$ represents the animal body weight at each measurement.

8.2.6 Data Analysis

The original data of measurement and observation must be recorded. The analysis and processing are based on the original data, and the results are presented as mean and standard error of the mean (Mean ± SEM). T-test is used to analyze the difference in tumor volume between the control group and the treatment group. $P < 0.05$ indicates statistical significance.

**8.3 Test Results**

8.3.1 Tumor Inhibitory Effect of Test Drugs on PSN1 Subcutaneous Tumor Xenograft Mouse Model

As shown in Figure 6, after oral administration for 6 days, at the end of the experiment (day 31), compared with the vehicle group, each administration group could significantly ($p<0.0001$) inhibit the growth of mouse tumors. The body weight of the NMS-P937 60 mg/kg group decreased severely, with half of the mice died, resulting in the inability to statistically analyze TGI. The tumor growth inhibition rate ($TGI_{TV}$) of each group is shown in Table 30.

**Table 30. Tumor Growth Inhibition Rate ($TGI_{TV}$) of Each Group at the Endpoint of the Test**

| Treatment | Dose (mg/kg) | TGI (%) | Mouse Mortality Rate (%) |
|---|---|---|---|
| CPD04 | 90 | 40 | 0 |
| CPD06 | 60 | 53 | 0 |
| NMS-P937 | 60 | NA | 50% |

8.3.2 Safety of Test Drugs in PSN1 Subcutaneous Tumor Xenograft Mouse Model

As shown in Figure 7, during the treatment, 4 mice in the NMS-P937 60mg/kg group died on D27 and D31, respectively. At the end of the test, only two mice in the NMS-P937 60mg/kg group showed slight body weight recovery. The weight loss may be due to the poor tolerance of tumor-bearing mice to NMS-P937. No mice died in the CPD06 60mg/kg group with body weight loss not exceeding 15% during administration, and the body weight recovered after drug withdrawal. In the CPD04 90mg/kg group, no mouse death was found, and no body weight loss was observed throughout the period.

**8.4 Test Conclusion**

In conclusion, in the PSN1 subcutaneous tumor xenograft mouse model, CPD06 at 60 mg/kg and CPD04 at 90 mg/kg significantly inhibited tumor growth in mice. The tumor growth inhibition effect of the CPD06 60 mg/kg administration group was significantly superior to that of other treatment groups, with $TGI_{TV}$ reaching 53%. The toxicity of NMS-P937 at 60 mg/kg in highly immune-deficient mice was far greater than that of CPD06 at 60 mg/kg and CPD04 at 90 mg/kg, with 4 out of 8 mice dying in the NMS-P937 60 mg/kg group.

The foregoing description is considered to be merely an illustration of the principles of the present disclosure. In addition, since many modifications and variations will be apparent to those skilled in the art, it is not intended to limit the present disclosure to the exact configuration and process as described above. Therefore, all suitable modifications and equivalents may be considered to fall within the scope of the present disclosure as defined by the appended claims.

All publications, patents, and patent applications cited herein are hereby incorporated by reference into this disclosure in their entirety.

## Claims

1. A stereoisomer, deuterated derivative or stereoisomeric deuterated derivative of a compound of Formula (I):

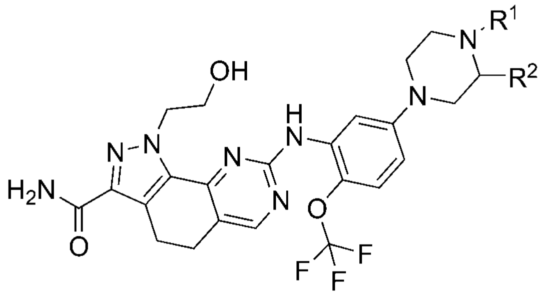

(I)

or a pharmaceutically acceptable salt, solvate thereof,
wherein $R^1$ and $R^2$ are each independently selected from hydrogen, deuterium, deuterated or non-deuterated $C_{1-6}$ alkyl; or $R^1$ and $R^2$ together with the N atom and the C atom to which they are attached form an optionally substituted, deuterated or non-deuterated 5-membered heterocyclic ring;

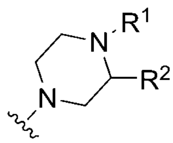

is optionally

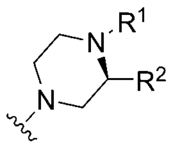

or

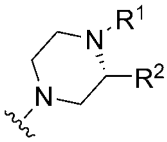

;

and/or

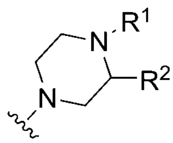

is optionally substituted with one or more deuterium atoms.

2. The stereoisomer, deuterated derivative or stereoisomeric deuterated derivative of a compound of Formula (I), or a pharmaceutically acceptable salt, solvate thereof according to claim 1, wherein

   $R^1$ is a deuterated or non-deuterated $C_{1-6}$ alkyl, preferably methyl or deuterated methyl, more preferably perdeuterated methyl; and/or
   $R^2$ is hydrogen or deuterium.

3. The stereoisomer, deuterated derivative or stereoisomeric deuterated derivative of a compound of Formula (I), or a pharmaceutically acceptable salt, solvate thereof according to claim 1, wherein

   $R^1$ and $R^2$ together with the N atom and C atom to which they are attached form an optionally substituted 5-membered heterocyclic ring as follows:

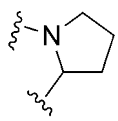

,

wherein the 5-membered heterocyclic ring is optionally substituted with 1-7 deuterium atoms.

**4.** The stereoisomer, deuterated derivative or stereoisomeric deuterated derivative of a compound of Formula (I), or a pharmaceutically acceptable salt, solvate thereof according to claim 3, wherein

$R^1$ and $R^2$ together with the N atom and C atom to which they are attached form an optionally substituted 5-membered heterocyclic ring selected from:

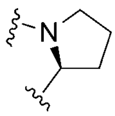 or 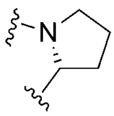 ,

wherein the 5-membered heterocyclic ring is optionally substituted with 1-7 deuterium atoms.

**5.** The stereoisomer, deuterated derivative or stereoisomeric deuterated derivative of a compound of Formula (I), or a pharmaceutically acceptable salt, solvate thereof according to any one of the preceding claims, wherein

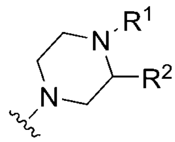

is optionally substituted with 1-8 deuterium atoms.

**6.** The stereoisomer, deuterated derivative or stereoisomeric deuterated derivative of a compound of Formula (I), or a pharmaceutically acceptable salt, solvate thereof according to claim 1, wherein the stereoisomer, deuterated derivative or stereoisomeric deuterated derivative of a compound of Formula (I) is selected from:

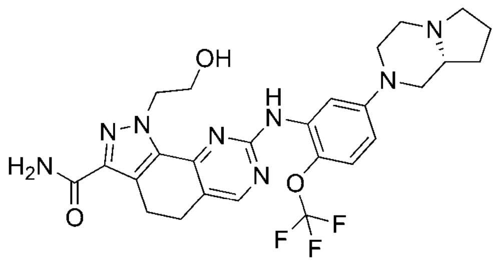 ,

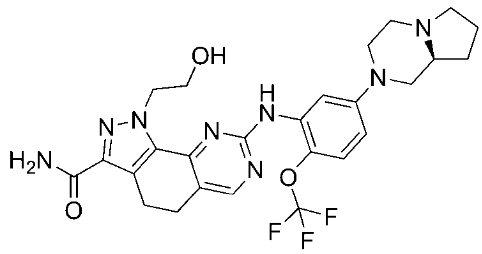 ,

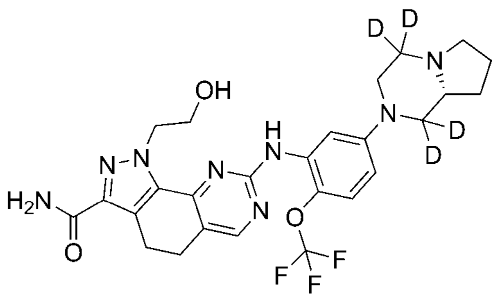 ,

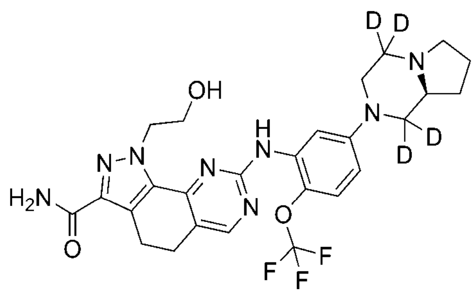 ,

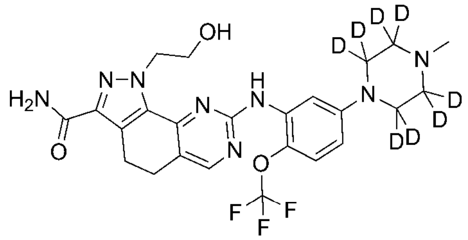

and

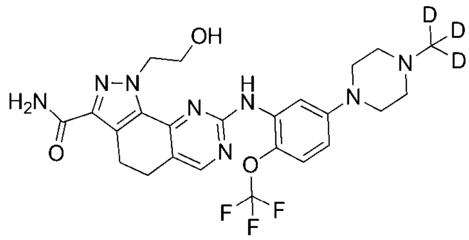

.

**7.** A pharmaceutical composition, comprising the stereoisomer, deuterated derivative or stereoisomeric deuterated derivative of a compound of Formula (I), or a pharmaceutically acceptable salt, solvate thereof according to any one of claims 1 to 6, and a pharmaceutically acceptable carrier or excipient.

**8.** A method for treating diseases and conditions caused by dysregulated PLK1 activity and/or diseases and conditions associated with PLK1 in a subject in need thereof, the method comprising administering to the subject a therapeutically effective amount of the stereoisomer, deuterated derivative or stereoisomeric deuterated derivative of a compound of Formula (I), or a pharmaceutically acceptable salt, solvate thereof according to any one of claims 1 to 6.

**9.** The method according to claim 8, wherein the diseases and conditions caused by dysregulated PLK1 activity and/or diseases and conditions associated with PLK1 are selected from the group consisting of leukemia, lymphoma, pancreatic cancer, breast cancer, prostate cancer, lung cancer, ovarian cancer, colorectal cancer, liver cancer, gastric cancer, esophageal cancer, melanoma, multiple myeloma, and sepsis.

**10.** The stereoisomer, deuterated derivative or stereoisomeric deuterated derivative of a compound of Formula (I), or a pharmaceutically acceptable salt, solvate thereof according to any one of claims 1 to 6, for use in treating diseases and conditions caused by dysregulated PLK1 activity and/or diseases and conditions associated with PLK1.

**11.** The stereoisomer, deuterated derivative or stereoisomeric deuterated derivative of a compound of Formula (I), or a pharmaceutically acceptable salt, solvate thereof, for use in treating diseases and conditions caused by dysregulated PLK1 activity and/or diseases and conditions associated with PLK1 according to claim 10, wherein the diseases and conditions caused by dysregulated PLK1 activity and/or diseases and conditions associated with PLK1 are selected from the group consisting of leukemia, lymphoma, pancreatic cancer, breast cancer, prostate cancer, lung cancer, ovarian cancer, colorectal cancer, liver cancer, gastric cancer, esophageal cancer, melanoma, multiple myeloma, and sepsis.

**12.** Use of the stereoisomer, deuterated derivative or stereoisomeric deuterated derivative of a compound of Formula (I), or a pharmaceutically acceptable salt, solvate thereof according to any one of claims 1 to 6, in the preparation of a medicament for treating diseases and conditions caused by dysregulated PLK1 activity and/or diseases and conditions associated with PLK1.

**13.** The use according to claim 12, wherein the diseases and conditions caused by dysregulated PLK1 activity and/or diseases and conditions associated with PLK1 are selected from the group consisting of leukemia, lymphoma, pancreatic cancer, breast cancer, prostate cancer, lung cancer, ovarian cancer, colorectal cancer, liver cancer, gastric cancer, esophageal cancer, melanoma, multiple myeloma, and sepsis.

**14.** A kit for treating diseases and conditions caused by dysregulated PLK1 activity and/or diseases and conditions associated with PLK1, the kit comprising

the stereoisomer, deuterated derivative or stereoisomeric deuterated derivative of a compound of Formula (I), or a pharmaceutically acceptable salt, solvate thereof according to any one of claims 1 to 6, or the pharmaceutical composition according to claim 7;
a container; and
optionally a package insert or label indicating treatment.

**15.** A kit according to claim 14, wherein the diseases and conditions caused by dysregulated PLK1 activity and/or diseases and conditions associated with PLK1 are selected from the group consisting of leukemia, lymphoma, pancreatic cancer, breast cancer, prostate cancer, lung cancer, ovarian cancer, colorectal cancer, liver cancer, gastric cancer, esophageal cancer, melanoma, multiple myeloma, and sepsis.

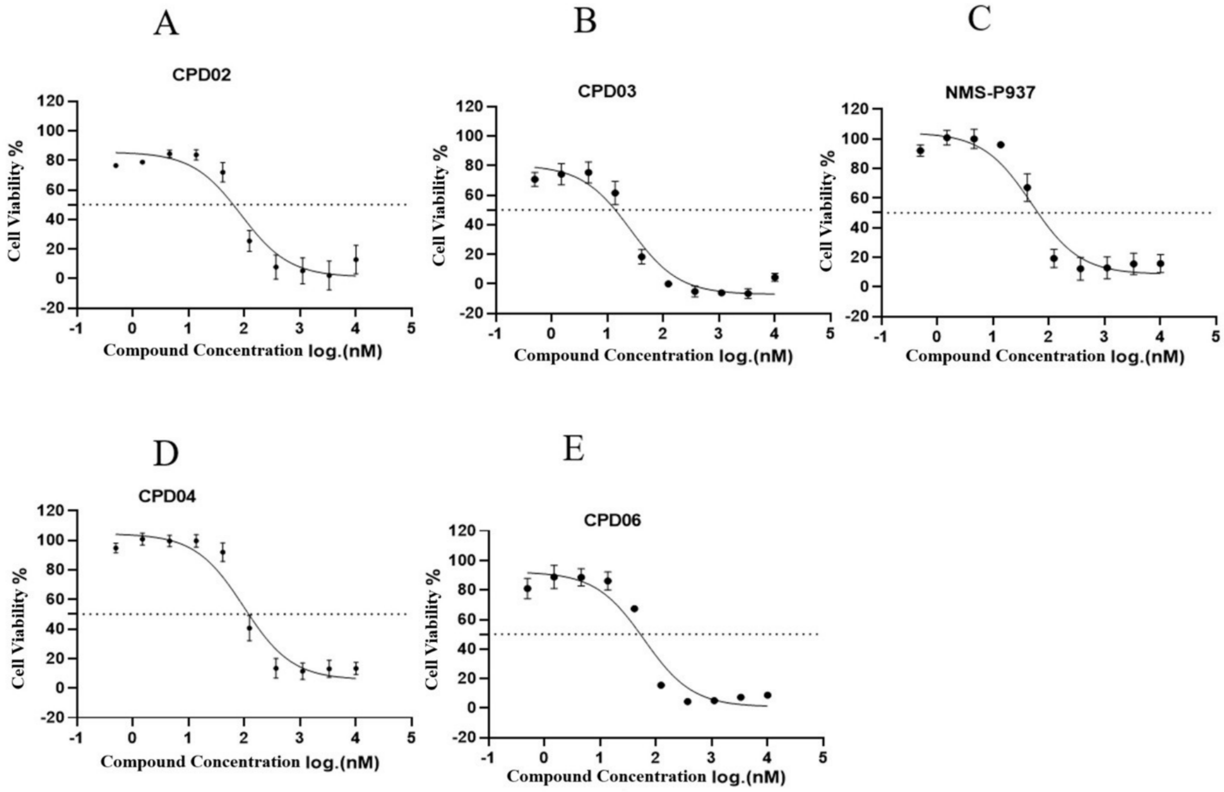
A
CPD02
B
CPD03
C
NMS-P937
D
CPD04
E
CPD06
Cell Viability %
Compound Concentration log.(nM)
120
100
80
60
40
20
0
-20
-1
0
1
2
3
4
5

FIG. 1

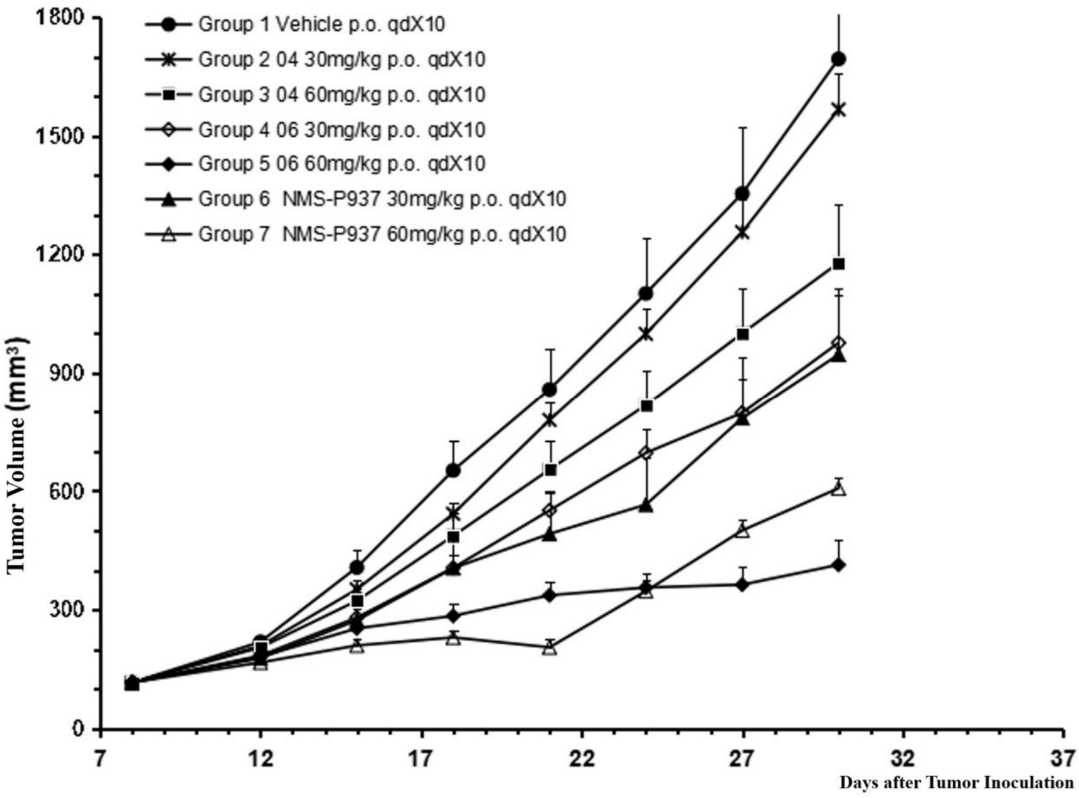
Group 1 Vehicle p.o. qdX10
Group 2 04 30mg/kg p.o. qdX10
Group 3 04 60mg/kg p.o. qdX10
Group 4 06 30mg/kg p.o. qdX10
Group 5 06 60mg/kg p.o. qdX10
Group 6 NMS-P937 30mg/kg p.o. qdX10
Group 7 NMS-P937 60mg/kg p.o. qdX10
Tumor Volume (mm³)
1800
1500
1200
900
600
300
0
7
12
17
22
27
32
37
Days after Tumor Inoculation

FIG. 2

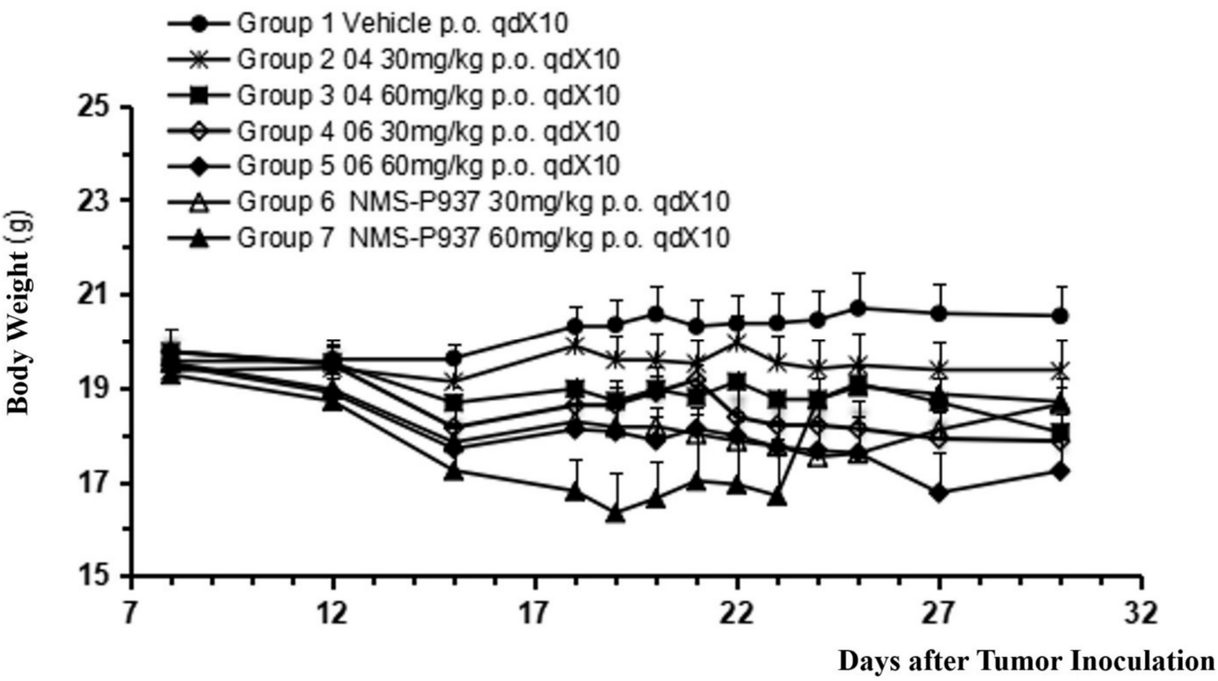
Group 1 Vehicle p.o. qdX10
Group 2 04 30mg/kg p.o. qdX10
Group 3 04 60mg/kg p.o. qdX10
Group 4 06 30mg/kg p.o. qdX10
Group 5 06 60mg/kg p.o. qdX10
Group 6 NMS-P937 30mg/kg p.o. qdX10
Group 7 NMS-P937 60mg/kg p.o. qdX10
Body Weight (g)
25
23
21
19
17
15
7
12
17
22
27
32
Days after Tumor Inoculation

FIG. 3

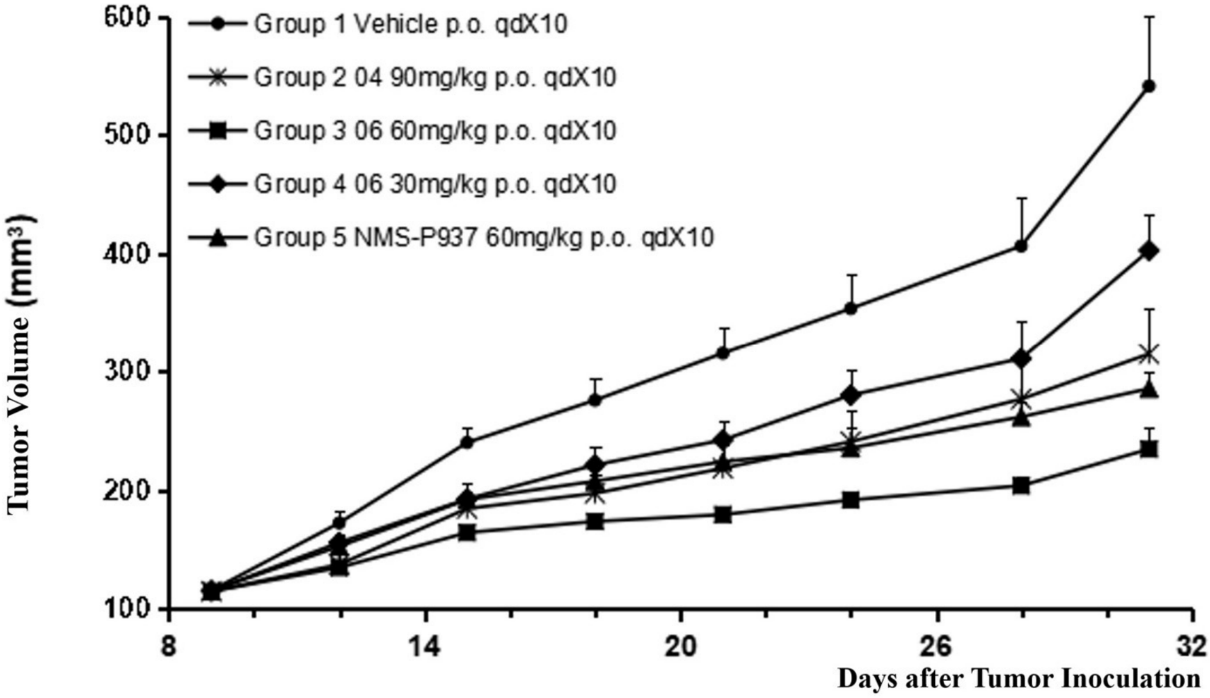
Group 1 Vehicle p.o. qdX10
Group 2 04 90mg/kg p.o. qdX10
Group 3 06 60mg/kg p.o. qdX10
Group 4 06 30mg/kg p.o. qdX10
Group 5 NMS-P937 60mg/kg p.o. qdX10
Tumor Volume (mm³)
600
500
400
300
200
100
8
14
20
26
32
Days after Tumor Inoculation

FIG. 4

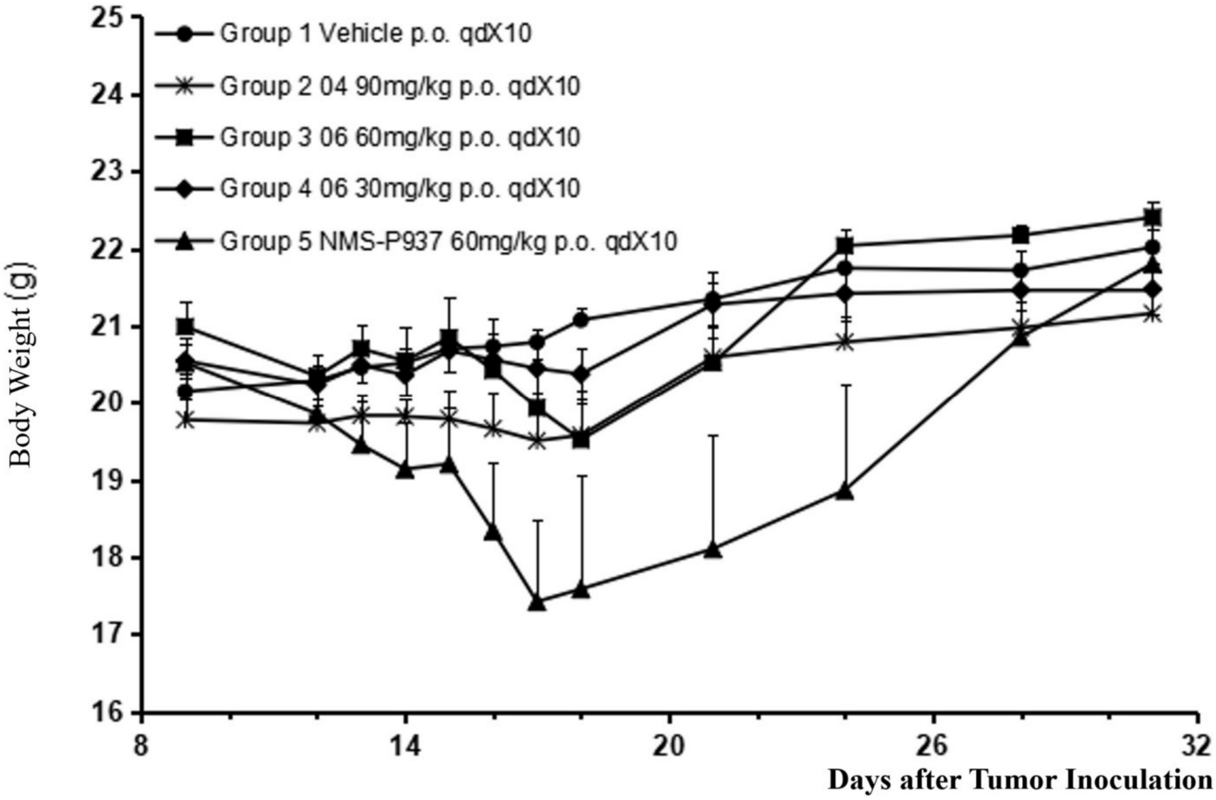
Group 1 Vehicle p.o. qdX10
Group 2 04 90mg/kg p.o. qdX10
Group 3 06 60mg/kg p.o. qdX10
Group 4 06 30mg/kg p.o. qdX10
Group 5 NMS-P937 60mg/kg p.o. qdX10
Body Weight (g)
25
24
23
22
21
20
19
18
17
16
8
14
20
26
32
Days after Tumor Inoculation

FIG. 5

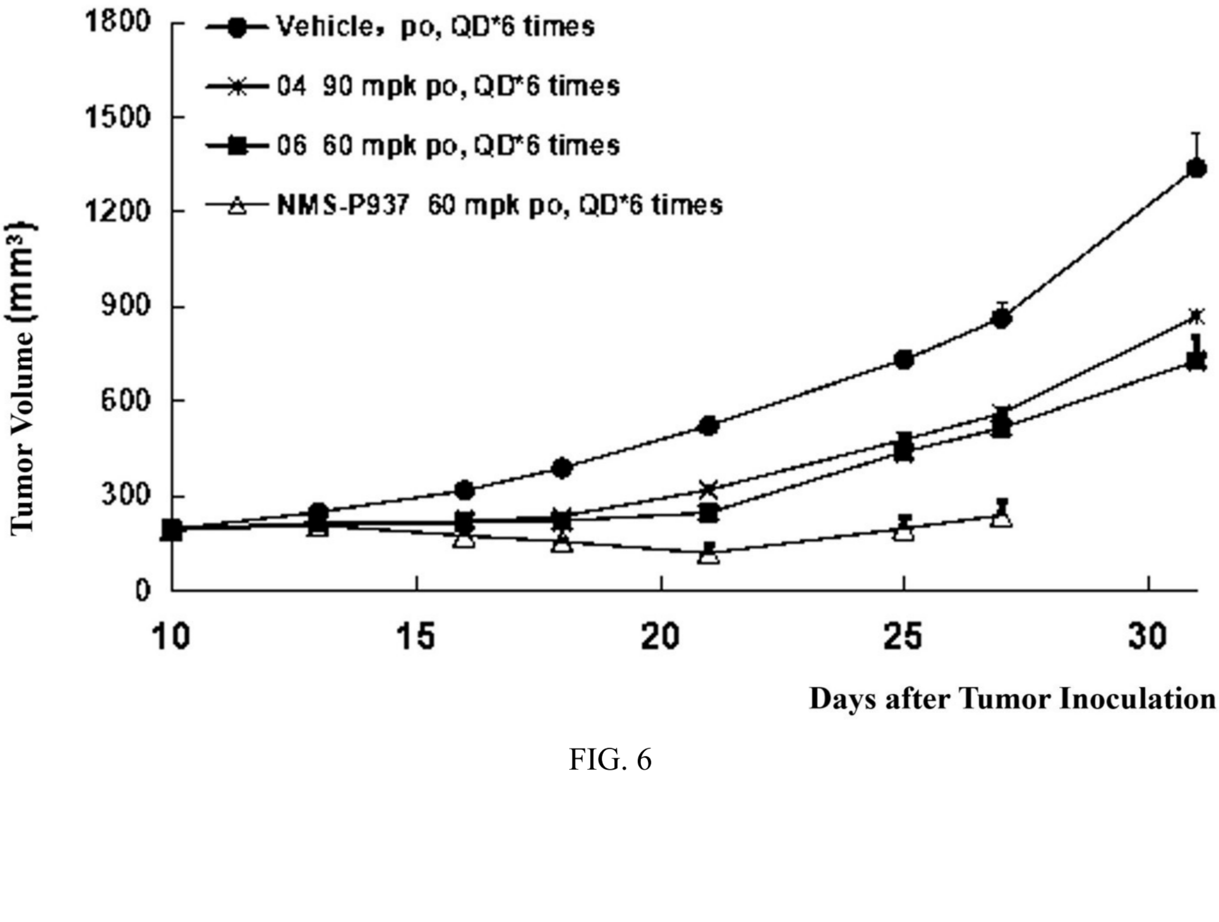
Vehicle, po, QD*6 times
04 90 mpk po, QD*6 times
06 60 mpk po, QD*6 times
NMS-P937 60 mpk po, QD*6 times
Tumor Volume (mm³)
1800
1500
1200
900
600
300
0
10
15
20
25
30
Days after Tumor Inoculation

FIG. 6

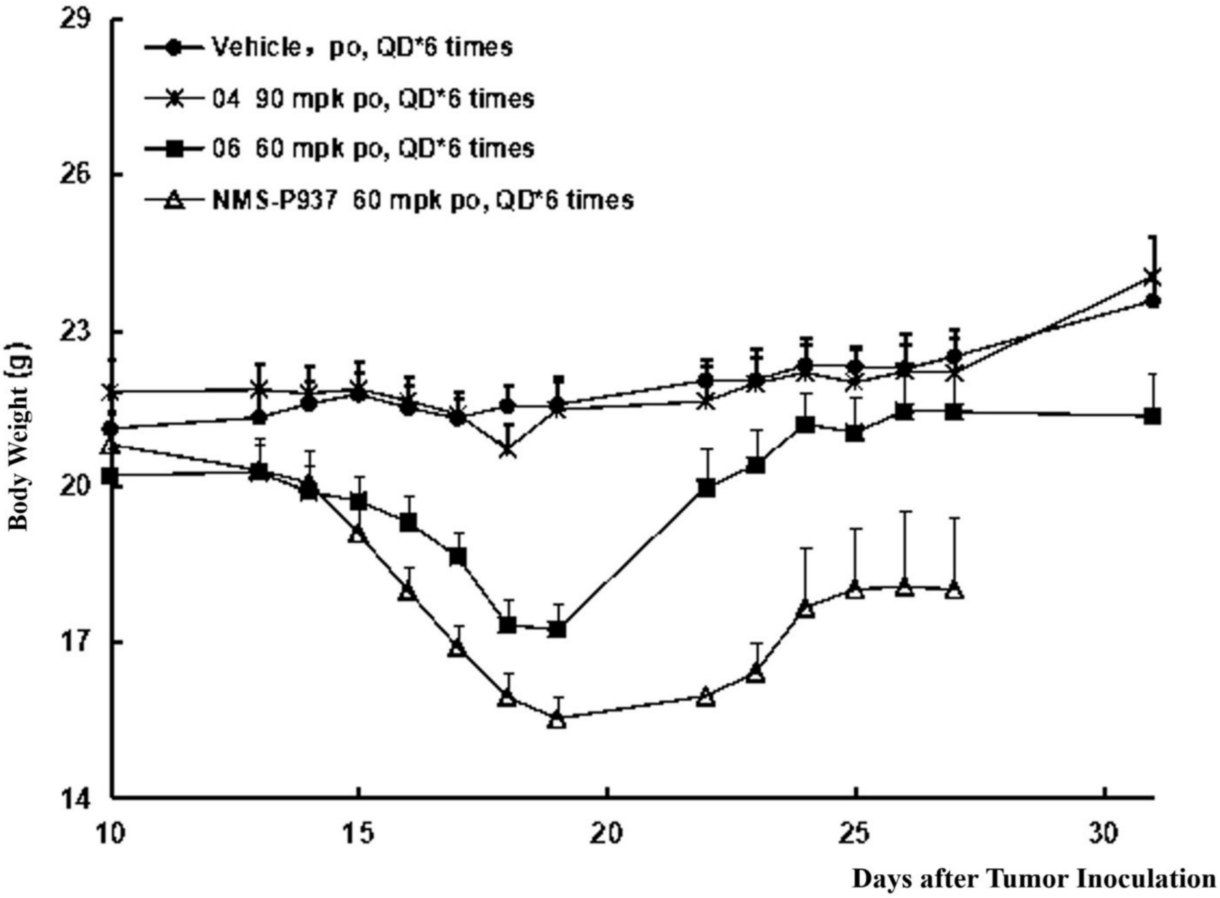

Vehicle，po, QD*6 times
04 90 mpk po, QD*6 times
06 60 mpk po, QD*6 times
NMS-P937 60 mpk po, QD*6 times
Body Weight (g)
29
26
23
20
17
14
10
15
20
25
30
Days after Tumor Inoculation


FIG. 7

## INTERNATIONAL SEARCH REPORT

International application No.
**PCT/CN2024/072704**

**A. CLASSIFICATION OF SUBJECT MATTER**

C07D495/04(2006.01)i; C07D487/14(2006.01)i; A61P31/12(2006.01)i; A61P37/00(2006.01)i; A61K31/517(2006.01)i; A61K31/519(2006.01)i; A61P25/00(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

CNKI, CNABS, CNTXT, VCN, VEN, ENTXT, ENTXTC, STN(REG, CAPLUS): PLK, 抑制剂, 癌, 瘤, inhibitor, cancer, tumor, 结构式, structural formula

**C. DOCUMENTS CONSIDERED TO BE RELEVANT**

| Category* | Citation of document, with indication, where appropriate, of the relevant passages | Relevant to claim No. |
|---|---|---|
| A | CN 101563351 A (NERVIANO MEDICAL SCIENCES S.R.L.) 21 October 2009 (2009-10-21)<br>entire document | 1-7, 12-15 |
| A | CN 102079746 A (NERVIANO MEDICAL SCIENCES S.R.L.) 01 June 2011 (2011-06-01)<br>entire document | 1-7, 12-15 |
| A | CN 113784969 A (UPPTHERA) 10 December 2021 (2021-12-10)<br>entire document | 1-7, 12-15 |
| A | CN 114685520 A (WUHAN REPUTATION-REPUTATION MEDICAL SCIENCE AND TECHNOLOGY LIMITED COMPANY) 01 July 2022 (2022-07-01)<br>entire document | 1-7, 12-15 |
| X | WO 2011012534 A1 (NERVIANO MEDICAL SCIENCES SRL et al.) 03 February 2011 (2011-02-03)<br>claims 1 and 6-12, and description, page 1, and embodiments 1-5 | 1-7, 12-15 |
| X | WO 2022143576 A1 (NEWSOARA BIOPHARMA CO., LTD.) 07 July 2022 (2022-07-07)<br>claims 1-3 and 29-32, and description, embodiment 2 | 1-7, 12-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 April 2024** | **29 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/072704

C. DOCUMENTS CONSIDERED TO BE RELEVANT

| Category* | Citation of document, with indication, where appropriate, of the relevant passages | Relevant to claim No. |
|---|---|---|
| PX | WO 2023104178 A1 (SHANDONG LUYE PHARMACEUTICAL CO., LTD.) 15 June 2023 (2023-06-15)<br>claims 1-14 | 1-7, 12-15 |
| PX | LU, Jing et al. "Design, Synthesis, and Biological Evaluation of Novel Molecules as Potent Inhibitors of PLK1"<br>*Bioorganic Chemistry*, No. no. 139, 13 July 2023 (2023-07-13),<br>pp. 1-15 | 1-7, 12-15 |

Form PCT/ISA/210 (second sheet) (July 2022)

**INTERNATIONAL SEARCH REPORT**

International application No.
**PCT/CN2024/072704**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8-11**
because they relate to subject matter not required to be searched by this Authority, namely:

Claims 8-11 relate to a method for treatment of the human/animal body (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/072704**

| Patent document cited in search report | Publication date (day/month/year) | Patent family member(s) | Publication date (day/month/year) |
|---|---|---|---|
| CN 101563351 A | 21 October 2009 | UA 102219 C2 | 25 June 2013 |
| | | ZA 200904912 A | 29 September 2010 |
| CN 102079746 A | 01 June 2011 | US 2017050972 A1 | 23 February 2017 |
| | | US 9637497 B2 | 02 May 2017 |
| | | RS 52899 B | 28 February 2014 |
| | | US 2015158876 A1 | 11 June 2015 |
| | | US 9464090 B2 | 11 October 2016 |
| | | BR 122019010200 B1 | 15 December 2020 |
| | | BR 122019010200 B8 | 27 July 2021 |
| | | US 2007185143 A1 | 09 August 2007 |
| | | US 7482354 B2 | 27 January 2009 |
| | | MY 142019 A | 16 August 2010 |
| | | HRP 20050967 A2 | 31 May 2007 |
| | | HRP 20050967 B1 | 28 March 2014 |
| | | HRP 20050967 B8 | 25 April 2014 |
| | | US 2013338148 A1 | 19 December 2013 |
| | | US 8981089 B2 | 17 March 2015 |
| | | GEP 20094664 B | 10 April 2009 |
| | | CR 8102 A | 09 August 2006 |
| | | US 2019194214 A1 | 27 June 2019 |
| | | ECSP 056194 A | 19 April 2006 |
| | | SI 1636236 T1 | 31 January 2014 |
| | | TW 200505924 A | 16 February 2005 |
| | | TWI 349672 B | 01 October 2011 |
| | | EP 1636236 A1 | 22 March 2006 |
| | | EP 1636236 B1 | 09 October 2013 |
| | | OA 13170 A | 13 December 2006 |
| | | JP 2007502851 A | 15 February 2007 |
| | | JP 5043432 B2 | 10 October 2012 |
| | | AU 2004240772 A1 | 02 December 2004 |
| | | AU 2004240772 B2 | 28 April 2011 |
| | | BRPI 0410563 A | 20 June 2006 |
| | | BRPI 0410563 B1 | 01 December 2020 |
| | | BRPI 0410563 B8 | 25 May 2021 |
| | | US 2021300935 A1 | 30 September 2021 |
| | | TNSN 05298 A1 | 10 July 2007 |
| | | MXPA 05012573 A | 22 February 2006 |
| | | CY 1114708 T1 | 05 October 2016 |
| | | NZ 543661 A | 31 May 2009 |
| | | MEP 25908 A | 10 June 2010 |
| | | ME 00142 B | 10 October 2010 |
| | | WO 2004104007 A1 | 02 December 2004 |
| | | RS 20050944 A | 05 June 2008 |
| | | ES 2436524 T3 | 02 January 2014 |
| | | UA 80763 C2 | 25 October 2007 |
| | | AR 044543 A1 | 21 September 2005 |
| | | IS 8132 A | 18 November 2005 |
| | | IS 2939 B | 15 March 2016 |
| | | PT 1636236 E | 16 December 2013 |
| | | NO 20055496 D0 | 21 November 2005 |

Form PCT/ISA/210 (patent family annex) (July 2022)

**INTERNATIONAL SEARCH REPORT**
**Information on patent family members**

International application No.
**PCT/CN2024/072704**

| Patent document cited in search report | | | Publication date (day/month/year) | Patent family member(s) | | | Publication date (day/month/year) |
|---|---|---|---|---|---|---|---|
| | | | | NO | 20055496 | L | 14 February 2006 |
| | | | | NO | 334992 | B1 | 18 August 2014 |
| | | | | ZA | 200509486 | B | 25 September 2008 |
| | | | | EA | 200501849 | A1 | 30 June 2006 |
| | | | | EA | 010904 | B1 | 30 December 2008 |
| | | | | IL | 172046 | A | 30 March 2017 |
| | | | | DK | 1636236 | T3 | 09 December 2013 |
| | | | | US | 2017190714 | A1 | 06 July 2017 |
| | | | | US | 10280176 | B2 | 07 May 2019 |
| | | | | PL | 1636236 | T3 | 28 February 2014 |
| | | | | CO | 5721006 | A2 | 31 January 2007 |
| | | | | AR | 102722 | A2 | 22 March 2017 |
| | | | | CA | 2526578 | A1 | 02 December 2004 |
| | | | | CA | 2526578 | C | 24 January 2012 |
| | | | | AP | 200503452 | A0 | 31 December 2005 |
| | | | | AP | 2005003452 | A0 | 30 October 2009 |
| | | | | US | 2009124605 | A1 | 14 May 2009 |
| | | | | US | 8541429 | B2 | 24 September 2013 |
| | | | | KR | 20060015294 | A | 16 February 2006 |
| | | | | KR | 101084871 | B1 | 21 November 2011 |
| CN | 113784969 | A | 10 December 2021 | KR | 20210150341 | A | 10 December 2021 |
| | | | | KR | 102529222 | B1 | 08 May 2023 |
| | | | | KR | 102313752 | B1 | 19 October 2021 |
| | | | | KR | 102313753 | B1 | 19 October 2021 |
| | | | | EP | 3911654 | A1 | 24 November 2021 |
| | | | | EP | 3911654 | A4 | 11 May 2022 |
| | | | | US | 2023159515 | A1 | 25 May 2023 |
| | | | | US | 2023104076 | A1 | 06 April 2023 |
| | | | | US | 11833208 | B2 | 05 December 2023 |
| | | | | KR | 20210122164 | A | 08 October 2021 |
| | | | | JP | 2023171659 | A | 01 December 2023 |
| | | | | KR | 20210122162 | A | 08 October 2021 |
| | | | | KR | 102337029 | B1 | 09 December 2021 |
| | | | | KR | 102319264 | B1 | 01 November 2021 |
| | | | | US | 2023158158 | A1 | 25 May 2023 |
| | | | | WO | 2021194318 | A1 | 30 September 2021 |
| | | | | WO | 2021194319 | A1 | 30 September 2021 |
| | | | | WO | 2021194320 | A1 | 30 September 2021 |
| | | | | US | 2023203006 | A1 | 29 June 2023 |
| | | | | KR | 20210122163 | A | 08 October 2021 |
| | | | | WO | 2021194321 | A1 | 30 September 2021 |
| | | | | KR | 20210122165 | A | 08 October 2021 |
| | | | | US | 2023372498 | A1 | 23 November 2023 |
| | | | | JP | 2022539579 | A | 12 September 2022 |
| | | | | JP | 7440940 | B2 | 29 February 2024 |
| CN | 114685520 | A | 01 July 2022 | None | | | |
| WO | 2011012534 | A1 | 03 February 2011 | CN | 102470136 | A | 23 May 2012 |
| | | | | TW | 201109334 | A | 16 March 2011 |
| | | | | US | 2012157468 | A1 | 21 June 2012 |
| | | | | EP | 2459196 | A1 | 06 June 2012 |

Form PCT/ISA/210 (patent family annex) (July 2022)

**INTERNATIONAL SEARCH REPORT**
**Information on patent family members**

International application No.
**PCT/CN2024/072704**

| Patent document cited in search report | | | Publication date (day/month/year) | Patent family member(s) | | | Publication date (day/month/year) |
|---|---|---|---|---|---|---|---|
| | | | | JP | 2013500299 | A | 07 January 2013 |
| | | | | AR | 077422 | A1 | 24 August 2011 |
| WO | 2022143576 | A1 | 07 July 2022 | CN | 116528871 | A | 01 August 2023 |
| | | | | TW | 202241413 | A | 01 November 2022 |
| WO | 2023104178 | A1 | 15 June 2023 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310083163X
- CN 202310430237


### Non-patent literature cited in the description


- *Translational Oncology Volume*, 16 February 2022, 101332
- *Bioorg Med Chem Lett.*, 15 May 2011, vol. 21 (10), 2969-74
- *Bioorg Med Chem Lett.*, 15 November 2010, vol. 20 (22), 6489-94
- *Mol Cancer Ther*, April 2012, vol. 11 (4)
- Organic Chemistry. Thomas Sorrell, University Science Books, 1999
- **SMITH** ; **MARCH**. March's Advanced Organic Chemistry. John Wiley & Sons, Inc., 2001
- **LAROCK**. Comprehensive Organic Transformations. VCH Publishers, Inc., 1989
- **CARRUTHERS**. Some Modem Methods of Organic Synthesis. Cambridge University Press, 1987
- **STAHL** ; **WERMUTH**. Handbook of Pharmaceutical Salts: Properties, Selection and Use. Wiley-VCH, 2002
- Pro-drugs as Novel Delivery Systems. **T. HIGUCHI** ; **W. STELLA**. ACS Symposium Series, vol. 14
- Bioreversible Carriers in Drug Design. Pergamon Press, 1987
- *Reference can also be made to Nature Reviews/Drug Discovery*, 2008, vol. 7, 355
- *Current Opinion in Drug Discovery and Development*, 2007, vol. 10, 550
- **H. BUNDGAARD**. Design of Prodrugs. Elsevier, 1985
- Designing Prodrugs and Bioprecursors. **Y. M. CHOI-SLEDESKI** ; **C. G. WERMUTH**. Practice of Medicinal Chemistry. Elsevier, 2015, 657-696
- **ANSEL** ; **HOWARD C et al.** Ansel's Pharmaceutical Dosage Forms and Drug Delivery Systems. Lippincott, Williams & Wilkins, 2004
- **GENNARO** ; **ALFONSO R. et al.** Remington: The Science and Practice of Pharmacy. Lippincott, Williams & Wilkins, 2000
- **ROWE** ; **RAYMOND C**. Handbook of Pharmaceutical Excipients. Pharmaceutical Press, 2005
- **HOOVER** ; **JOHN E.** Remington's Pharmaceutical Sciences. Mack Publishing Co., 1975
- Pharmaceutical Dosage Forms. Marcel Decker, 1980
- Handbook of Pharmaceutical Excipients. American Pharmaceutical Association, 1999
- Comprehensive Organic Chemistry. Elsevier
- **RICHARD LAROCK**. Comprehensive Organic Transformations: A Guide to Functional Group Preparation. John Wiley and Sons
- Compendium of Organic Synthesis Methods. Wiley-Interscience, vol. I-XII
- **T.W. GREENE** ; **P.G.M. WUTS**. Protective Groups in Organic Synthesis. John Wiley & Sons